(19) Europäisches Patentamt / European Patent Office / Office européen des brevets

(11) **EP 4 272 645 A1**

(12) **EUROPEAN PATENT APPLICATION**
published in accordance with Art. 153(4) EPC

(43) Date of publication:
**08.11.2023 Bulletin 2023/45**

(21) Application number: **21915854.0**

(22) Date of filing: **29.12.2021**

(51) International Patent Classification (IPC):
*A61B 5/346* (2021.01)    *A61B 5/327* (2021.01)
*A61B 5/00* (2006.01)    *G16H 50/20* (2018.01)
*G06N 3/08* (2023.01)    *G06N 3/04* (2023.01)

(52) Cooperative Patent Classification (CPC):
**A61B 5/00; A61B 5/327; A61B 5/346; G06N 3/04;
G06N 3/08; G16H 50/20**

(86) International application number:
**PCT/KR2021/020227**

(87) International publication number:
**WO 2022/146057 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.12.2020 KR 20200186808**

(71) Applicant: **Seoul National University Hospital
Seoul 03080 (KR)**

(72) Inventor: **KIM, Joonghee
Seongnam-si, Gyeonggi-do 13596 (KR)**

(74) Representative: **V.O.
P.O. Box 87930
2508 DH Den Haag (NL)**

(54) **METHOD AND APPARATUS FOR CONVERTING ELECTRICAL BIOSIGNAL DATA INTO NUMERICAL VECTORS, AND METHOD AND APPARATUS FOR ANALYZING DISEASE BY USING SAME**

(57) Embodiments relate to a method and apparatus for extracting numerical vector information by using a deep learning algorithm from electrical biosignal data, particularly, single-channel or multi-channel electrocardiogram signals, or black-and-white or color electrocardiogram images obtained on the basis of the electrocardiogram signals, and to a method and apparatus for analyzing and predicting a disease, and providing diagnostic auxiliary information about the disease by using the method and apparatus.

FIG. 1

**Description**

[Technical Field]

The present specification relates to a method and apparatus for converting electrical biosignal data, particularly electrocardiogram data, into numerical vectors, and to a method and apparatus for using the data to analyze, predict a disease, and provide diagnostic auxiliary information regarding a disease.

[Background Art]

Electrical biosignals are signals in which changes in biosignals are represented by changes in electrical signals, representatively such as electroencephalogram (EEG), electromyogram (EMG), electrocoulography (EOG), and electrocardiogram (ECG). Since these electrical biosignals are unstructured data, they are not easily interpretable or utilizable.

Among those, in particular, an electrocardiogram (ECG) is a recording of the action current generated by the myocardium as the heart beats. The electrocardiogram is a simple test that takes less than a minute to perform and is used to diagnose acute myocardial infarction and arrhythmias. The electrocardiogram analysis is well established for the study of a patient's heart function and the identification of disorders of the heart, and doctors have been using an electrocardiogram (ECG) system to monitor the patient's heart activity for decades.

However, the electrocardiogram contains a lot of information that is difficult for even experts to interpret, and this information can be used to predict a wider range of patients' future conditions and appropriate treatments, but is currently in limited use.

[Disclosure]

[Technical Problem]

In one aspect, exemplary embodiments of the present application is directed to providing a method and apparatus that is capable of providing auxiliary information for analyzing, predicting, or diagnosing disease by extracting numerical information from electrical biosignal data, particularly electrocardiogram (ECG) data, and utilizing the numerical information within a clinical framework or fusing the numerical information with other information on a patient to maximize the range of applications.

[Technical Solution]

Exemplary embodiments of the present application provide an apparatus for converting electrical biosignal data into numerical vectors including: an acquisition unit configured to acquire electrical biosignal data; and an encoder configured to calculate numerical vectors by receiving the electrical biosignal data using a deep learning algorithm.

In addition, another exemplary embodiments of the present application provide an apparatus for analyzing a disease including: an analysis unit that performs to provide auxiliary information regarding disease-related analysis, prediction, or diagnosis using the encoder and the numerical vector described above.

In addition, still another exemplary embodiments of the present application provide a method of converting electrical biosignal signal, in particular, electrocardiogram data into numerical vectors, performed by a processor and comprising: acquiring electrical biosignal data; inputting the electrical biosignal data into an encoder; and calculating numerical vectors using a deep learning algorithm through the encoder.

In addition, still another exemplary embodiments of the present application provide a method of analyzing a disease, the method including: performing the steps described above, and further generating analytical results for providing auxiliary information regarding disease-related analysis, prediction, or diagnosis using the numerical vectors.

Specifically, in one aspect, an apparatus for converting electrical biosignal data into numerical vectors or an apparatus for analyzing a disease includes: an acquisition unit configured to acquire electrical biosignal data; and an encoder configured to calculate a first numerical vector by receiving the electrical biosignal data using a deep learning algorithm, and the first numerical vector is structured data associated with features extracted from the electrical biosignal data, which includes, in particular, contextually one or more of anatomic (positional) features and temporal features that can be extracted from the electrical biosignal data. The first numerical vector is effectively used for downstream tasks or machine learning, as described below.

The apparatus of an exemplary embodiment further includes an analysis unit that performs to provide disease or health-related analysis, prediction, or diagnosis-related auxiliary information using the first numerical vector.

The apparatus of one exemplary embodiment includes a plurality of downstream processing units that simultaneously process a plurality of tasks using the first numerical vector. Error signals from each of downstream task network output terminals may be back-propagated and gathered at an end terminal of a single encoder to train a single encoder, thereby improving universality of the first numerical vector.

In the apparatus of one exemplary embodiment, the first numerical vector may be concatenated with itself or with other structured data information to be used as the input numerical vector of the downstream processing unit.

In the apparatus of one exemplary embodiment, the encoder may be two or more, and a plurality of first numerical vectors output from each encoder may be concatenated to provide a single input numerical vector.

In the apparatus of one exemplary embodiment, N sequential electrical biosignal data may be passed through a single encoder to provide N sequential first numerical vectors.

The apparatus of one exemplary embodiment may provide an analysis, diagnosis, or prediction of a particular disease based on result values for each time point obtained by dividing the electrical biosignal data into certain time intervals and passing information from each divided data section through the encoder or the encoder and a downstream processing unit, or a weighted average for each time point of the corresponding result values for each time point.

The apparatus of one exemplary embodiment may fix network weights of the encoder in training a network of the downstream task and then modify (update) a network weight of the downstream task through the training, and further modify (update) an entire weight of the network of the encoder and the network of the downstream task through additional training.

In the apparatus of one exemplary embodiment, each of the plurality of downstream processing units may be performed by a multi-layer perceptron (MLP) having two or more fully connected layers.

In the apparatus of one exemplary embodiment, the MLP may be trained through a multi-task learning jointly with an encoding network training of the encoder, or may be trained separately after the encoder is trained first.

In the apparatus of one exemplary embodiment, the MLP receives additional structured data input information that is different from the first numerical vector, in which the additional structured data input information comprises at least one of age, gender, biosignals, numerical test results, natural language data information transformed into structured data, and structured data information converted from a different kind of biosignal data than the electrical biosignal data acquired from an acquisition unit, in which the biosignals comprise one or more biosignals selected from a group consisting of blood pressure, pulse rate, body temperature, respiratory rate, and oxygen saturation. The additional structured data entry information may be either concatenated with the first numerical vector or input separately from the first numerical vector.

The apparatus of one exemplary embodiment may further include a display unit configured to represent marginal probability of a particular disease occurrence in consideration of the acquired electrical biosignal data, together with marginal probability of a particular disease occurrence not in consideration of the acquired electrical biosignal data as a baseline risk probability when outputting the MLP, and configured to display how many times the marginal probability of a particular disease occurrence in consideration of the acquired electrical biosignal data is increased in ratio terms than the marginal probability of a particular disease occurrence not in consideration of the acquired electrical biosignal data, in case that the MLP predicts whether a particular disease occurs.

In the apparatus of one exemplary embodiment, the deep learning algorithm of the encoder may be based on a CNN and may include an encoder subunit.

In the apparatus of one exemplary embodiment, the encoder subunit may include: one or more convolution layers; one or more fully connected layers, where the fully connected layer comprises a nonlinear activation function; and a concentration layer configured to summarize feature sets extracted from the electrical biosignal data per channel to extract a representative value for each, and recalibrate the feature set per channel to reflect contribution of the feature set per channel on the basis of the representative values, in which the feature set may include features related to anatomical structures for each channel, and in which the recalibrated feature set per channel may be more focused on anatomical features for each channel compared to the feature set.

In the apparatus of one exemplary embodiment, the one or more convolution layers may include a depthwise-separable convolution layer configured to individually perform a convolution of electrical biosignal data for each of the one or more channels.

In the apparatus of one exemplary embodiment, the concentration layer may perform a pooling process on the feature set for summarizing the feature set.

In the apparatus of one exemplary embodiment, the concentration layer may pass the representative value for each channel through the fully connected layer to calculate a contribution for each channel, and the contribution for each channel may be multiplied by the feature set to recalibrate the feature set for each channel.

In the apparatus of one exemplary embodiment, the concentration layer may calculate the contribution per each channel by scaling a result of passing the representative value per each channel through the fully connected layer to a numerical value between a specific range.

In an apparatus of one exemplary embodiment, the encoder subunit includes a squeeze-excitation layer that extracts an average for each channel to produce a single scalar value, the scalar value for each channel is between 0 and 1, scaled according to the importance of the channel, and the vector of scalar values for each channel is passed to a fully connected layer, which may apply a sigmoid/RELU function to reduce the dimensionality.

In an apparatus of one exemplary embodiment, the encoder may be a plurality of convolution blocks, and the subunits may be included in a remaining convolution blocks except for a first conversion layer.

In an apparatus of one exemplary embodiment, the convolution block may include: a first encoder subunit; and a second encoder subunit, in which the first encoder subunit may be applied closer to an input terminal of the convolution block than an output terminal thereof compared to the second encoder subunit, and the concentration layer may focus more on the operation of summarizing the feature set to extract the representative value between the operation of summarizing the feature set extract the representative value and the operation of recalibrating according to the contribution of each channel compared to the second encoder subunit. - the features related to the anatomical structure are reflected more in the representative values of the first encoder subunit compared to the representative values of the second encoder subunit, and in which the second encoder subunit may be applied closer to the output terminal of the convolution block than the input terminal thereof compared to the first encoder subunit, and the concentration layer may focus more on the operation of recalibrating according to the contribution of each channel compared to the first encoder subunit between the operation of summarizing the feature set to extract the representative value and the operation of recalibrating according to the contribution of each channel.

In an apparatus of one exemplary embodiment, a final convolution block of the encoder may further include a non-local network, and the non-local network may implement spatial attention by comparing similarity between spatial points of the electrical biosignal data.

In an apparatus of one exemplary embodiment, the electrical biosignal data may be a signal of a single-channel or multi-channel, and the electrical biosignal signals input to the encoder may be in the form of a two-dimensional array of $C \times M$ (the number of each input lead (channel) X the number of measured values for each channel).

In an apparatus of one exemplary embodiment, the electrical biosignal data may be an electrical biosignal image, in which the electrical biosignal image may depict the electrical biosignal signals of all channels on a two-dimensional plane, in which the electrical biosignal image may be converted to an image that includes all of the lead channels or to an image per lead channel that is cropped per lead channel, and in which the electrical biosignal image input to the encoder may be converted into a black-and-white image of one or more lead channels or a three-dimensional array of $C \times W \times H$ (number of channels $\times$ number of horizontal pixels $\times$ number of vertical pixels) with three channels of R(Red), G(Green), and B(Blue) and input to the encoder.

In an apparatus of one exemplary embodiment, the analysis results may include a disease prediction, in which the electrical biosignal data may be the electrical biosignal data of the single-channel, when the analysis unit predicts disease, in which the disease may include shock, respiratory failure, cardiac arrest, acute coronary syndrome, myocardial infarction, and hyperkalemia, when the electrical biosignal data is electrocardiogram (ECG) data.

In the apparatus of an exemplary embodiment, the analysis results may include disease diagnostic auxiliary information to determine whether the disease has improved or worsened using the first numerical vector. When the analysis unit provides the disease diagnostic auxiliary information, the electrical biosignal data may be a plurality of electrical biosignal data measured at regular intervals, each of the plurality of electrical biosignal data is passed through a pooling layer of the encoder to provide the diagnostic auxiliary information of whether the disease has improved or worsened from the acquired first numerical vector.

In the apparatus of an exemplary embodiment, the analysis results may include a provision of the disease diagnostic auxiliary information, in which the electrical biosignal data may be a plurality of electrical biosignal data measured at regular or irregular time intervals, and in which the analysis unit may be trained to determine whether a particular disease has improved or worsened over time by arranging each of the first numerical vectors of the plurality of electrical biosignal data into sequential vectors, and concatenating the sequential vectors in a lengthwise direction of the vectors to pass the sequential vectors through the multilayer perceptron (MLP) network, or concatenating the sequential vectors in a vertical direction of the vector length to pass the sequential vectors through a transformer network, or sequentially passing the sequential vectors through an RNN without concatenating the sequential vectors to extract a second numerical vector, and by being on the basis of the extracted second numerical vector. In this case, the vector input for training the analysis unit may be a vector that concatenates a value encoding information on time with the first numerical vector.

In the apparatus of an exemplary embodiment, the encoder may be trained through a self-supervised learning on the basis of clinically defined morphological characteristics among characteristics of the electrical biosignal data.

In the apparatus of an exemplary embodiment, the encoder may be trained by a self-supervised learning using electrical biosignal data transformed in a particular way as training data.

In the apparatus of an exemplary embodiment, the encoder may be trained by an unsupervised learning, using the augmented electrical biosignal data as training data, and in which the encoder network may include a process of inputting each of the augmented electrical biosignal data having the original electrical biosignal data in common to the encoder, and calibrating each of the calculated first numerical vectors to be identical or have a high degree of similarity.

In the apparatus of an exemplary embodiment, the process of calibrating each of the calculated first numerical vectors to be identical or have a high degree of similarity may be to minimize a distance of each of the calculated first numerical vectors.

In the apparatus of an exemplary embodiment, the apparatus may be concatenated with a smart watch, a medical device or exercise equipment equipped with an electrical biosignal measurement device or a device with a smartphone app or an electronic health record system.

In an exemplary embodiment, the electrical biosignal in the above apparatus is preferably, in particular, an electrocardiogram (ECG).

Meanwhile, in another aspect, in a method of converting electrical biosignal data into a numerical vector, performed by a processor, or a method of analyzing a disease from electrical biosignal data using deep learning, performed by a processor, the method includes: acquiring electrical biosignal data; inputting the electrical biosignal data to an encoder; and using the encoder to calculate a first numerical vector using a deep learning algorithm, in which the first numerical vector may be structured data associated with features extracted from the electrical biosignal data, including, in particular, contextually, one or more of anatomical (positional) features and temporal features that may be extracted from the electrical biosignal data. The first numerical vector is effectively used for downstream tasks or machine learning, as described below.

In an exemplary embodiment, the method further includes performing to provide disease or health-related analysis, prediction, or diagnosis-related auxiliary information using the first numerical vector.

In an exemplary embodiment, the method may include simultaneously processing a plurality of downstream tasks using the first numerical vector. Error signals from each of downstream task network output terminals may be back-propagated and gathered at an end terminal of a single encoder to train a single encoder, thereby improving universality of the first numerical vector.

In the method of one exemplary embodiment, the first numerical vector may be concatenated with itself or with additional structured data information to be used as an input vector of the processing of downstream task.

In the method of one exemplary embodiment, the encoder may be two or more, and a plurality of first numerical vectors output from each encoder may be concatenated to provide a single input numerical vector.

In the method of one exemplary embodiment, N sequential electrical biosignal data may be passed through a single encoder to obtain N sequential first numerical vectors.

In the method of one exemplary embodiment, the method may provide an analysis, diagnosis, or prediction of a particular disease based on result values for each time point obtained by dividing the electrical biosignal data into certain time intervals and passing information from each divided data section through the encoder or the encoder and a downstream processing, or based on a weighted average for each time point of the corresponding result values for each time point.

In the method of one exemplary embodiment, network weights of the encoder may be fixed in training a network of the downstream task and then a network weight of the downstream task is modified (updated) through the training, and an entire weight of the network of the encoder and the network of the downstream task may be further modified (updated) through additional training.

In the method of one exemplary embodiment, each of the plurality of downstream task processing may be performed by a multi-layer perceptron (MLP) having two or more fully connected layers.

In the method of one exemplary embodiment, the MLP may be trained through a multi-task learning jointly with an encoding network training of the encoder, or may be trained separately after the encoder is trained first.

In the method of one exemplary embodiment, the MLP may receive additional structured data input information that is different from the first numerical vector, in which the additional structured data input information may include at least one of age, gender, biosignals, numerical test results, natural language data information transformed into structured data, and structured data information converted from a different kind of biosignal data than the electrical biosignal data acquired from an acquisition unit, in which the biosignals may include one or more biosignals selected from a group consisting of blood pressure, pulse rate, body temperature, respiratory rate, and oxygen saturation. The additional structured data entry information may be either concatenated with the first numerical vector or input separately from the first numerical vector.

In the method of one exemplary embodiment, the method may represent marginal probability of a particular disease occurrence in consideration of the acquired electrical biosignal data, together with marginal probability of a particular disease occurrence not in consideration of the acquired electrical biosignal data as a baseline risk probability when outputting the MLP, and display how many times the marginal probability of a particular disease occurrence in consideration of the acquired electrical biosignal data is increased in ratio terms than the marginal probability of a particular disease occurrence not in consideration of the acquired electrical biosignal data, in case that the MLP predicts whether a particular disease occurs.

In the method of one exemplary embodiment, the deep learning algorithm of the encoder may be based on a CNN and may include an encoder subunit.

In the method of one exemplary embodiment, the encoder subunit may include: one or more convolution layers;

one or more fully connected layers, where the fully connected layer comprises a nonlinear activation function; and a concentration layer configured to summarize feature sets extracted from the electrical biosignal data per channel to extract a representative value for each, and recalibrate the feature set per channel to reflect contribution of the feature set per channel on the basis of the representative values, in which the feature set may include features related to anatomical structures for each channel, and in which the recalibrated feature set per channel may be more focused on anatomical features for each channel compared to the feature set.

In the method of one exemplary embodiment, the one or more convolution layers may include a depthwise-separable convolution layer configured to individually perform a convolution of electrical biosignal data for each of the one or more channels.

In the method of one exemplary embodiment, the concentration layer may perform a pooling process on the feature set for summarizing the feature set.

In the method of one exemplary embodiment, the concentration layer may pass the representative value for each channel through the fully connected layer to calculate a contribution for each channel, and the contribution for each channel may be multiplied by the feature set to recalibrate the feature set for each channel.

In the method of one exemplary embodiment, the concentration layer may calculate the contribution per each channel by scaling a result of passing the representative value per each channel through the fully connected layer to a numerical value between a specific range.

In the method of one exemplary embodiment, the encoder subunit includes a squeeze-excitation layer that extracts an average for each channel to produce a single scalar value, the scalar value for each channel is between 0 and 1, scaled according to the importance of the channel, and the vector of scalar values for each channel is passed to a fully connected layer, which may apply a sigmoid/RELU function to reduce the dimensionality.

In the method of one exemplary embodiment, the encoder may be a plurality of convolution blocks, and the subunits may be included in a remaining convolution blocks except for a first conversion layer.

In the method according to one exemplary embodiment, the convolution block may include: a first encoder subunit; and a second encoder subunit, in which the first encoder subunit may be applied closer to an input terminal of the convolution block than an output terminal thereof compared to the second encoder subunit, and the concentration layer may focus more on the operation of summarizing the feature set to extract the representative value between the operation of summarizing the feature set to extract the representative value and the operation of recalibrating according to the contribution of each channel compared to the second encoder subunit. - the features related to the anatomical structure are reflected more in the representative values of the first encoder subunit compared to the representative values of the second encoder subunit, and in which the second encoder subunit may be applied closer to the output terminal of the convolution block than the input terminal thereof compared to the first encoder subunit, and the concentration layer may focus more on the operation of recalibrating according to the contribution of each channel compared to the first encoder subunit between the operation of summarizing the feature set to extract the representative value and the operation of recalibrating according to the contribution of each channel.

In the method of one exemplary embodiment, a final convolution block of the encoder may further include a non-local network, and the non-local network may implement spatial attention by comparing similarity between spatial points of the electrical biosignal data.

In the method of one exemplary embodiment, the electrical biosignal data may be a signal of a single-channel or multi-channel, and the electrical biosignal signals input to the encoder may be in the form of a two-dimensional array of $C \times M$ (the number of each input lead (channel) X the number of measured values for each channel).

In the method of one exemplary embodiment, the electrical biosignal data may be an electrical biosignal image, in which the electrical biosignal image may depict the electrical biosignal signals of all channels on a two-dimensional plane, in which the electrical biosignal image may be converted to an image that includes all of the lead channels or to an image per lead channel that is cropped per lead channel, and in which the electrical biosignal image input to the encoder may be converted into a black-and-white image of one or more lead channels or a three-dimensional array of $C \times W \times H$ (number of channels $\times$ number of horizontal pixels $\times$ number of vertical pixels) with three channels of R(Red), G(Green), and B(Blue) and input to the encoder.

In the method of one exemplary embodiment, the analysis results may include a disease prediction, in which the electrical biosignal data may be the electrical biosignal data of the single-channel, when the analysis unit predicts disease, in which the disease may include shock, respiratory failure, cardiac arrest, acute coronary syndrome, myocardial infarction, and hyperkalemia, when the electrical biosignal data is electrocardiogram (ECG) data.

In the method of one exemplary embodiment, the analysis results may include disease diagnostic auxiliary information to determine whether the disease has improved or worsened using the first numerical vector. When the disease diagnostic auxiliary information is provided, the electrical biosignal data may be a plurality of electrical biosignal data measured at regular intervals, each of the plurality of electrical biosignal data may be passed through a pooling layer of the encoder to provide the diagnostic auxiliary information of whether the disease has improved or worsened from the acquired first numerical vector.

In the method of one exemplary embodiment, the analysis results include providing disease diagnostic auxiliary information, and the electrical biosignal data is a plurality of electrical biosignal data measured at regular or irregular time intervals. The analysis unit may be trained to determine whether a particular disease has improved or worsened over time by arranging each of the first numerical vectors of the plurality of electrical biosignal data into sequential vectors, and concatenating the sequential vectors in a lengthwise direction of the vectors to pass the sequential vectors through the multilayer perceptron (MLP) network, or concatenating the sequential vectors in a vertical direction of the vector length to pass the sequential vectors through a transformer network, or sequentially passing the sequential vectors through an RNN without concatenating the sequential vectors to extract a second numerical vector, and by being on the basis of the extracted second numerical vector. In this case, the vector input for training the analysis unit may be a vector that concatenates a value encoding information on time with the first numerical vector.

In the method of one exemplary embodiment, the encoder may perform the training through a self-supervised learning on the basis of clinically defined morphological characteristics among characteristics of the electrical biosignal data.

In the method of one exemplary embodiment, the encoder may perform the training by a self-supervised learning using electrical biosignal data transformed in a particular way as training data.

In the method of one exemplary embodiment, the encoder may train through an unsupervised learning, using the augmented electrical biosignal data as training data, and in which the encoder network may include a process of inputting each of the augmented electrical biosignal data having the original electrical biosignal data in common to the encoder, and calibrating each of the calculated first numerical vectors to be identical or have a high degree of similarity.

In the method of one exemplary embodiment, the process of calibrating each of the calculated first numerical vectors to be identical or have a high degree of similarity may be to minimize a distance of each of the calculated first numerical vectors.

In the method of one exemplary embodiment, the electrical biosignal in the above apparatus is preferably, in particular, an electrocardiogram (ECG).

Meanwhile, in still another aspect, one exemplary embodiment provides a computer-readable recording medium that is readable by a computer and that stores program instructions operable by the computer. Here, when the program instructions are executed by a processor of the aforementioned computer, the processor performs the methods of the embodiments described above.

[Advantageous Effects]

According to exemplary embodiments of the present application, structured numerical vectors can be extracted from unstructured electrical biosignals, particularly electrocardiogram data, and utilized in a variety of clinical situations.

In particular, it is possible to utilize existing clinical frameworks as they are, but extract universal numerical information that can maximize the range of applications of electrical biosignal information, especially ECG information. This universal numerical information (embedding vector) can be used on its own as well as fused with other information on the patient. In addition, by quantifying electrical biosignal data, especially electrocardiogram data, changes in patient's condition can be easily quantified. Accordingly, the quantification of electrical biosignal data can be applied to triage in emergency rooms or disaster relief sites, and it can be applied to various healthcare equipment. In addition, structured numerical vectors can be added to existing artificial intelligence algorithms to facilitate the assessment and prediction of changes in a patient's condition and to provide auxiliary information on acute myocardial infarction or arrhythmia classification, as well as various types of diagnoses that may involve electrical biosignals, especially electrocardiogram, related to the heart, lungs, blood flow, etc.

The effects of the present application are not limited to the aforementioned effects, and other effects, which are not mentioned above, will be clearly understood by those skilled in the art from the claims.

[Description of Drawings]

In order to more clearly describe the exemplary embodiments of the present application, the drawings required for the description of the embodiments are briefly introduced below. It should be understood that the following drawings are for the purpose of describing embodiments of the present specification and are not intended to be limiting. In addition, for clarity of descriptions, some elements may be illustrated in the drawings below with various variations, including exaggeration and omission.

FIG. 1 is a schematic view illustrating an apparatus for converting electrocardiogram data into numerical vectors to analyze disease, according to an embodiment of the present application.
FIG. 2 is a flowchart of a method of converting electrocardiogram data into numerical vectors to analyze a disease, according to an embodiment of the present application.

FIG. 3 is a view illustrating an ECG encoder subunit, according to an embodiment of the present application.

FIG. 4 is a view illustrating an ECG encoder according to an embodiment of the present application.

FIG. 5 is a view illustrating a utilization of numerical vectors obtained from a plurality of electrocardiogram data obtained through repeated measurements, according to another embodiment of the present application.

FIG. 6 is a view illustrating a utilization of N sequentially obtained numerical vectors, according to still another embodiment of the present application.

[Mode for Disclosure]

Hereinafter, some embodiments of the present application will be described in detail with reference to the illustrative drawings. In assigning reference numerals to constituent elements of the respective drawings, the same constituent elements will be designated by the same reference numerals, if possible, even though the constituent elements are illustrated in different drawings. In addition, in the description of the present embodiments, the specific descriptions of publicly known related configurations or functions may be omitted when it is determined that the specific descriptions may obscure the subject matter of the present technical ideas.

## Terminology

The terms "comprise," "have," or "include" used in the present specification may mean that other constituent elements can be added unless these terms are used with the term "only". Unless otherwise particularly and clearly stated, the singular expressions of the constituent element used herein are intended to include the plural expressions.

In addition, the terms first, second, A, B, (a), and (b) may be used to describe constituent elements of the present application. Unless otherwise specified, these terms are used only for the purpose of discriminating one constituent element from another constituent element, and the nature, the sequences, the orders, or the number of the constituent elements are not limited by the terms.

In the present specification, the term " training" or " learning" refers to performing machine learning by computing according to a procedure.

In the present specification, a network refers to a neural network of a machine learning algorithm or model.

In the present specification, terms such as "unit," "module," "device," or "system" are intended to refer not only to hardware, but also to combinations of software driven by that hardware. For example, hardware can be a data processing device that includes a central processing unit (CPU), a graphics processing unit (GPU), or another processor. In addition, software can refer to a running process, an object, an executable, a thread of execution, a program, and the like.

In the present disclosure, a numerical vector or numerical vector information is a structured numerical coordinate with a consistent structural and/or meaningful form that is associated with (reflective of the corresponding features) electrical biosignal data, created through a deep learning algorithm for application to one or more machine learning tasks or tasks.

Converting a specific data to a numerical vector means, for example, electrocardiograms, turning unstructured data of various formats (the electrical signal itself or its output) and sizes (channel type, number, and measurement time) into a numerical vector (or an array) that is shorter than the original, has a constant length (or, in case of an array, a constant dimension and size), and where each element has a consistent meaning per position. This provides a consistent representation of where a particular electrocardiogram is located in the vector space defined by each element, and this abstract coordinate information can be utilized in a variety of ways (algorithms) by various downstream tasks.

The characteristics of the network structures presented in the present disclosure (in particular, network structures including squeeze-excitation, non-local networks) may allow these numerical vectors to be associated with features extracted from the electrical biosignal data, including anatomical (positional) features and/or temporal features per channel of the electrical biosignal data.

Taking an electrocardiogram as an example, the anatomical characteristics or anatomical spatial characteristics of the electrocardiogram signal ( a space that can be defined as a configuration of the anatomical positions of the electrodes in which the electrocardiogram is measured) and/or the temporal characteristics or temporal spatial characteristics ( a space that can be defined as an entire time interval in which the electrocardiogram signal is measured) can be broadly and efficiently reflected, and the characteristics of the training method (auxiliary learning based on multi-task learning) can efficiently extract highly universal features in multiple tasks during the broad feature extraction process as described above.

This greatly facilitates the training of new downstream tasks, enabling the extraction of high-quality numerical vectors that facilitate few-shot or one-shot learning.

In the present specification, anatomical (positional) features of an electrical biosignal or anatomical spatial features means features related to the anatomical position or structure (of a measurement target from which the electrical biosignal is measured) represented by the electrical biosignal of each channel or each lead.

Using an electrocardiogram as an example, each of the electrodes (leads) on an electrocardiogram represents a different anatomical position in the heart, and the interpretation of the morphological features found on each lead may be different. For example, Q waves indicate myocardial infarction, but are also commonly observed in normal in leads I, aVL, V5, and V6. In considering these anatomical positions, it is particularly important to note that when interpreting the characteristics of a particular location (lead), the characteristics of other positions must be contextually considered. For example, when the ST segment exhibits a characteristic of elevated ST segment at baseline in the precordial leads V1, V2, V3, etc., in order to quantify the clinical meaning of this characteristic (myocardial infarction), the reciprocal change (change in ST segment) observed in the inferior wall leads (II, III, aVF) should be taken into account to emphasize or attenuate the numerical reflection of this characteristic.

This contextual consideration, that is, the contextual consideration of the characteristics of other positions (leads) when interpreting the characteristics of a particular position (lead) of an electrical biosignal, may be implemented in exemplary embodiments of the present application through a squeeze-excitation network structure, which will be described later. In this respect, exemplary embodiments of the present application refer to anatomical (positional) features that may be extracted from electrical biosignal data being in particular contextually included.

In the present specification, temporal features or temporal-spatial features of an electrical biosignal refer to characteristics related to how morphological characteristics of the electrical biosignal are distributed within a one-dimensional time interval over which the electrical biosignal is measured.

For example, electrocardiograms are measured over various time ranges, as short as a few minutes, as long as a few hours, or as long as several days. In this variable one-dimensional space, ECG waveforms consisting of P, QRS, and T waves are placed at various frequencies, regularly or irregularly, depending on the patient's condition. When considering the characteristics of this placement, it is particularly important to note that the clinical interpretation of any characteristic within that one-dimensional space must take into account (contextual consideration) other characteristics that are farther away. For example, consider the diagnosis of a fatal arrhythmia called complete atrioventricular block. In general, arrhythmias use temporal characteristics related to how each characteristic is placed in the one-dimensional space mentioned above. In complete atrioventricular block, P waves and QRS waves are not synchronized, but appear at separate rates, and it is important in the diagnostic process to determine whether a small deflection of a particular QRS wave is a P wave superimposed on a QRS wave. In order to recognize this, it is important to determine which of the QRS deflections and various morphological features that are potentially P waves elsewhere are actually P waves, and this process is not possible without considering the context of the corresponding features in relation to each other.

Exemplary embodiments of the present application apply a non-local network structure to solve the problem of extracting temporal characteristics that take into account the context between these temporally separated characteristics. In this respect, exemplary embodiments of the present application refer to temporal features that may be extracted from electrical biosignal data being in particular contextually included.

In the present specification, numerical vectors may be represented separately, such as a first numerical vector, a second numerical vector, and the like. For example, the first numerical vector may refer to an output from an encoder using a deep learning algorithm, and the second numerical vector may refer to an output from an additional machine learning algorithm, such as a downstream task, using the first numerical vector. In some drawings, for example, sequential vectors included in the first numerical vector may be represented as vector 1, vector 2, vector 3, and so on.

In the present specification, embedding may refer to an operation that converts unstructured data, such as an electrocardiogram, into a numerical vector as described above, or to the output thereof (the numerical vector itself).

In the present specification, a numerical vector having universality means that the numerical vector can be used for other machine learning purposes, preferably multiple machine learning purposes, in addition to the specific purpose. That is, the numerical vector implies the morphological characteristics of a particular electrical biosignal (e.g., an electrocardiogram) in such a preferably comprehensive and/or efficient manner that the numerical vector can be effectively used in an unknown number of downstream tasks, preferably two or more downstream tasks, and more preferably most downstream tasks, which are already being applied or may be applied in the future.

For example, for ease of understanding, consider a non-universal numerical vector. Assuming that a numerical vector of 100 elements has characteristics that are effective in diagnosing a specific disease, for example, a myocardial infarction, with three elements, and the remaining 97 elements have redundant or noisy information, in this case, the numerical vector cannot be used for downstream tasks other than the diagnosis of a myocardial infarction and is not universal. In order to fill the elements of these vectors with meaningful information, it is possible to perform multiple clinical diagnostic tasks simultaneously, rather than a single diagnosis. However, this only ensures that the numerical vectors encode characteristics that are relevant to the diagnoses already trained, thus making it more difficult to apply to unknown downstream tasks.

In contrast, in exemplary embodiments of the present application, squeeze excitation, non-local networks, and the like can enhance the range and quality of characteristic information to be included in the numerical vector, thereby improving universality, as described above. Furthermore, in exemplary embodiments of the present application, the universality of numerical vectors can be further increased by further applying 1) supervised learning based on existing clinically defined morphological features, and 2) self-supervised learning to learn morphological features of electrocardiograms that are independent of clinical information. In addition, the universality of numerical vectors can be further increased by performing additional 3) unsupervised learning, which will be described below, for efficient placement of information in the vector space defined by numerical vectors.

In the present specification, unstructured data can refer to a set of measured numerical data that 1) has an inconsistent number and/or size of dimensions, 2) has an inconsistent interpretation of numbers across positions, or 3) has a large size or complexity that requires a simple transformation.

Structured data in the present specification, in contrast, means that the number of dimensions and size are constant, such that the interpretation of each numerical value is consistent with position, and the data is simple (without an excessive number of elements) and not large in size compared to unstructured data, such that it may be possible to train machine learning algorithms for downstream tasks with less data compared to unstructured data. For example, this may include an electrocardiogram that has been embedded and turned into a numerical vector, or tabular data such as a patient's age, gender, blood pressure, pulse rate, respiration rate, and temperature.

In the present specification, a downstream task may refer to one or more, in particular a plurality of machine learning tasks that use numerical vectors obtained through embedding. As described below, this may include 1) supervised learning, 2) unsupervised learning, 3) self-supervised learning, 4) clustering, and 5) anomaly detection.

In the present specification, a method of analyzing a disease or an apparatus for analyzing a disease has a meaning that includes analyzing, predicting, and providing diagnostic information on disease or health.

In the present specification, ECG refers to an electrocardiogram or electrocardiogram signal, which is a record of the heart's electrical activity in the form of a quantified waveform. More specifically, the waveform is composed of a vector sum of action potentials generated by the heart's special excitatory & conductive system. That is, the electrocardiogram signal is a signal of the vector sum of action potentials from each constituent element of the heart such as the sinoatrial node (SA node), atrioventricular node (AV node), His bundle, and furkinje fibers, measured from electrodes contacted in vitro. In general, two or more electrodes are used to measure an electrocardiogram, and the electrodes are paired. The ECG signal, electrocardiogram, or electrocardiogram signal, when used interchangeably, does not mean different things and may be used interchangeably.

## Description of exemplary embodiments

Exemplary embodiments of the present application use deep learning-based artificial intelligence algorithms to extract numerical vector information, particularly universal numerical vector information, which can be used in a variety of clinical situations, from single-channel or multi-channel electrical biosignal data, in particular single-channel or multi-channel electrocardiogram signals, or black-and-white or color electrocardiogram images obtained based on the electrocardiogram signals.

The obtained numerical vector information, in particular the numerical vector information obtained from the electrocardiogram, can be used to diagnose or predict, individually or collectively, whether 1) cardiac rhythm abnormalities (tachycardia, bradycardia, various arrhythmias), 2) cardiac structure and function abnormalities (heart failure, pericardial tamponade, stenosis/failure of valves, pulmonary arterial hypertension, pulmonary embolism, cardiomyopathy), 3) various clinical events (shock, respiratory failure, cardiac arrest, cardiogenic syncope and sudden death, acute coronary syndrome, myocardial infarction, hyperkalemia) and 4) various clinical interventions (such as administration of inotropes, ventilatory support, coronary angiography and blood transfusions) will occur or become necessary within a specified period of time.

In addition, other structured information (age, gender, blood pressure, pulse rate, respiratory rate, body temperature, numerical test results, etc.) and unstructured information (main symptoms, underlying diseases, text, radiological and ultrasound imaging information, acoustic information such as auscultatory sounds, and various biosignals) that are structured with appropriate transformations can be further concatenated into the corresponding numerical vectors to improve accuracy of diagnosis.

The algorithms of the exemplary embodiments of the present application may include a deep learning algorithm portion, such as a modified convolutional neural network (CNN), and/or an algorithm portion that processes additional information other than electrical biosignal data, such as electrocardiogram data.

In addition, exemplary the embodiments of the present application can acquire electrical biosignal data, such as electrocardiogram data, to provide auxiliary information for analyzing, predicting, and diagnosing a disease.

In the exemplary embodiments, an apparatus for converting electrical biosignal data into numerical vectors may include an acquisition unit that acquires electrical biosignal data; and an encoder that receives the electrical biosignal data as input and uses a deep learning algorithm to calculate a numerical vector (which may be referred to as a first numerical vector).

In the exemplary embodiments, an apparatus for analyzing a disease by converting electrical biosignal data into numerical vectors may include: an acquisition unit that acquires electrical biosignal data; an encoder that receives the electrical biosignal data as input and uses a deep learning algorithm to calculate a numerical vector (which may be referred to as a first numerical vector); and an analysis unit that provides disease-related analytical information, predictive information, or diagnostic auxiliary information using the numerical vector.

In the exemplary embodiments, a method of converting electrical biosignal data to a numerical vector, performed by a processor, may include: acquiring electrical biosignal data; inputting the electrical biosignal data to an encoder; and calculating a numerical vector (which may be referred to as a first numerical vector) using a deep learning algorithm through the encoder.

In the exemplary embodiments, a method of analyzing a disease from electrical biosignal data, performed by a processor and using deep learning, is provided, the method includes: acquiring electrical biosignal data; inputting the electrical biosignal data to an encoder; calculating a numerical vector (which may be referred to as a first numerical vector) using a deep learning algorithm through the encoder; and providing disease-related analytical, predictive, or diagnostic auxiliary information using the numerical vector.

In the exemplary embodiments, the electrical biosignal may be an electroencephalogram (EEG), an electromyogram (EMG), an electrocoulography (EOG), an electrocardiogram (ECG), and the like, with an electrocardiogram (ECG) being particularly preferred, as described below.

In the exemplary embodiments, the numerical vector can be used simultaneously for a downstream task.

As described above, since multiple tasks are configured to be performed simultaneously, the error signals from an output terminal of each downstream task network are back-propagated to an end terminal of the encoder to train a single encoder. Therefore, the numerical vector can be a numerical vector with increased universality.

In one exemplary embodiment, the first numerical vector may be used by itself or concatenated with additional structured data information as an input vector of the downstream task network.

Here, the additional structured data information may include at least one of age, gender, or vital signs such as blood pressure, pulse rate, body temperature, respiratory rate, oxygen saturation, laboratory test results, unstructured data [image, sound, biosignal, etc. (where the corresponding biosignal is a different biosignal than the electrical biosignal input to the encoder to obtain the first numerical vector)] converted into structured data information through machine learning methods, and natural language information such as symptoms, diagnoses, medical records, etc. converted into structured data through natural language processing.

In one exemplary embodiment, the encoder may be two or more, and a plurality of first numerical vectors output from each encoder may be concatenated to provide a single input numerical vector. The corresponding input numerical vector may be set as an input value of the downstream task network and the diagnosis to be predicted may be set as an output value of the downstream task network to train the network.

In one exemplary embodiment, N sequential electrical biosignal data may be passed through a single encoder to obtain N sequential first numerical vectors. These N sequential first numerical vectors can be used as input for training the downstream task network that predicts whether a particular disease will improve or worsen over time, or predicts risk of a particular disease, or predicts occurrence of a clinical event.

In one exemplary embodiment, the apparatus may provide an analysis, diagnosis, or prediction of a particular disease based on result values for each time point obtained by dividing the electrical biosignal data into certain time intervals and passing information from each divided data section through the encoder, or the encoder and a downstream processing, or a weighted average for each time point of the corresponding result values for each time point.

In one exemplary embodiment, the apparatus for converting numerical vectors or the apparatus for analyzing a disease includes a downstream processing unit or a downstream processing step that processes a downstream task using the numerical vectors, in which the downstream task may be to process a plurality of tasks, each of which may be performed by a multi-layer perceptron (MLP) having two or more fully connected layers.

In one exemplary embodiment, in case that the MLP predicts whether a particular disease occurs, the apparatus may represent marginal probability of a particular disease occurrence in consideration of the electrical biosignal data, together with marginal probability of the disease occurrence not in consideration of the electrical biosignal data as a baseline risk probability when outputting the MLP, and display how many times the marginal probability of the disease occurrence in consideration of the electrical biosignal data is increased in ratio terms than the marginal probability of the disease occurrence not in consideration of the acquired electrical biosignal data.

In one exemplary embodiment, the MLP for each task may be trained jointly with an encoding network of the encoder, or may be trained separately after the encoder is trained first.

In one exemplary embodiment, the encoder may be based on a CNN and include an encoder subunit, and the encoder subunit may include a depthwise-separable convolution layer that independently convolves the electrical biosignal data for each channel.

In one exemplary embodiment, the encoder subunit applies a squeeze-excitation mechanism to extract a single value (average or highest value) for each channel. The resulting numerical vector is then passed through a network consisting of two or more fully connected layers with a non-linear activation function, such as RELU, and a sigmoid function is applied to obtain a numerical value between 0-1 for each channel, which is then multiplied by the corresponding channel to recalibrate the features for each channel.

In one exemplary embodiment, the encoder may include a first convolution layer and a plurality of convolution blocks, each including a plurality of encoder subunits.

In one exemplary embodiment, a final convolution block of the encoder may further include a non-local network. The non-local network (or non-local neural network) uses the characteristics of all positions (spatial/temporal points) of the input data when encoding information of a particular position (spatial points for images, temporal points for electrocardiogram signals). In this process, each position has a different degree of contribution, which is determined by an attention mechanism.

In one exemplary embodiment, the MLP for each task may receive an additional structured data input information that is different from the numerical vector output by the ECG encoder. Here, the additional input information may include at least one of age, gender, or vital signs such as blood pressure, pulse rate, body temperature, respiratory rate, comorbidities, oxygen saturation, laboratory test results, and unstructured data (images, sounds, biosignals, etc.) converted into structured numerical information.

In exemplary embodiments, the aforementioned apparatus may be electrical biosignal measurement equipment or interpretation equipment. For example, the apparatus may be, but is not limited to, a smart watch, a device such as a medical device or exercise equipment such as a treadmill equipped with an electrical biosignal meter, or a device with an app installed, or an electronic health records (EHR) system that uses cameras or scanning equipment and is equipped with interpretation algorithms.

In addition, in exemplary embodiments, there is provided a computer-readable recording medium that is readable by a computer and that stores program instructions executable by the computer, in which the program instructions, when executed by a processor of the computer, cause the processor to perform the method of converting a numerical vector or the method of analyzing a disease as described above.

**Description of preferred embodiment**

As described above, the method and apparatus for converting electrical biosignal data into numerical vectors, and the method and apparatus for analyzing disease using the same, of the exemplary embodiments of the present application may be particularly well suited for use in the case of an electrocardiogram (ECG) among electrical biosignals, and therefore preferred embodiments will be described in more detail below, focusing on the electrocardiogram.

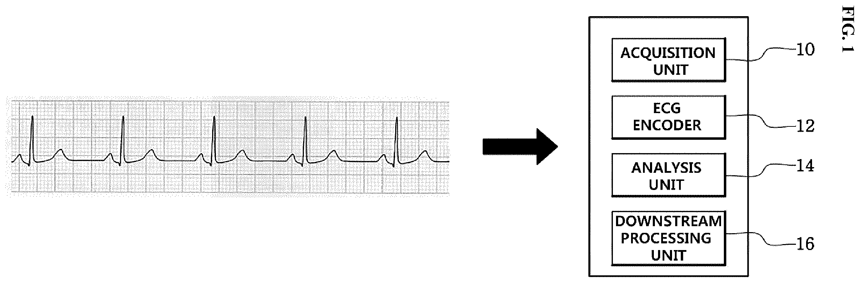

FIG. 1 is a schematic view illustrating an apparatus 1 (hereinafter referred to as the "disease analysis apparatus") that converts electrocardiogram data into numerical vectors to analyze a disease, according to an embodiment of the present application.

With reference to FIG. 1, a disease analysis apparatus 1 according to an embodiment of the present application includes an acquisition unit for acquiring electrocardiogram data from an electrocardiogram measurement device; an ECG encoder for receiving the electrocardiogram data and calculating a numerical vector using deep learning; an analysis unit for generating an analysis result using the numerical vector to perform a disease-related prediction or diagnosis; and a downstream processing unit 16. Although FIG. 1 illustrates the downstream processing unit 16 separately from the analysis unit 14, the downstream processing unit 16 may be included as part of the analysis unit 14 or may be a replacement for the analysis unit 14.

The acquisition unit 10 may directly or indirectly acquire an electrocardiogram signal from an electrocardiogram measurement device that is attached to a part of a body of a target and measures an electrocardiogram signal of the target (a user).

The acquisition unit 10 may also be connected to receive information from the electrocardiogram measurement device, which measures the electrocardiogram signal of the target through sensors attached to a part of the body of the target. Then, the acquisition unit 10 may directly acquire the electrocardiogram signal from the electrocardiogram measurement device.

The sensor may be attached to a part of the subject's body to measure the electrocardiogram signal of the target (a user). Electrocardiogram signals acquired from a sensor and an electrocardiogram signal measurement device may be converted to digital signals by an analog-to-digital converter (ADC). In addition, the electrocardiogram measurement device (not illustrated) may measure a vital sign when it is determined that the user's body is in contact with the touch panel for a predetermined amount of time. According to another embodiment, the acquisition unit 10 may acquire not only an electrocardiogram signal (raw signal), but also an electrocardiogram image that is output to paper or an image and visualized based on the previously obtained electrocardiogram signal.

The acquisition unit 10 may also be connected to receive information from the electrocardiogram measurement device, which measures the electrocardiogram signal of the target through sensors attached to a part of the body of the target. Then, the acquisition unit 10 may directly acquire the electrocardiogram signal from the electrocardiogram measurement device.

Alternatively, the acquisition unit 10 may be connected for wired or wireless electrical communication with an external device. Then, the acquisition unit 10 may acquire the electrocardiogram signal data pre-acquired or stored in the external device. The external device is either connected to the electrocardiogram measurement device or has acquired electrocardiogram signal data from another external device connected to the electrocardiogram measurement device. Therefore, the acquisition of the electrocardiogram signal data by the acquisition unit 10 from the external device may be treated as indirectly acquiring the electrocardiogram signal.

The ECG encoder 12 is a computing device including a processor, which receives electrocardiogram data from the acquisition unit 10 as input, analyzes the electrocardiogram signal to extract a feature map for each channel, and calculates a numerical vector through various computational processes. Thereafter, the numerical vector may be used to perform analysis, prediction, and diagnostic auxiliary information provision of various diseases through the analysis unit 14 or the downstream processing unit 16.

In an embodiment, the ECG encoder 12 may be a variety of computing devices, including, for example, a computer such as a personal computer (PC) or laptop, a smartphone, a server, and the like.

In an embodiment, the ECG encoder 12 may be implemented as a server, and electrocardiogram data input to the ECG encoder may be performed through a device (e.g., a user terminal or signal input equipment) connected to the server.

In this case, the server is a plurality of computer systems or a computer software implemented as a network server, which may provide a variety of information organized as a website. Here, a network server means a computer system and computer software (network server program) connected with subordinate devices capable of communicating with other network servers over a computer network, such as a private intranet or the Internet, which receives requests to perform tasks, performs the tasks, and provides results of the performance. However, in addition to these network server programs, it should be understood as a broader concept that includes a series of applications that run on the network server and, in some cases, various databases built therein. For example, in case of including various databases, the ECG encoder 12 may be configured to use external database information, such as a cloud, in which case the ECG encoder 12 may access and communicate data to an external database server (e.g., a cloud server) in accordance with the operation.

In an embodiment, the ECG encoder 12 for calculating numerical vectors may include a deep learning model, in which a large amount of unstructured ECG data is trained on a deep neural network consisting of multiple layers of networks to automatically learn the features of each electrocardiogram data and thereby train the network for calculating numerical vectors in a way that minimizes an error in an objective function, that is, prediction accuracy.

In an embodiment, the deep learning model included by the ECG encoder 12 may use, for example, a convolutional neural network (CNN), a deep hierarchical network (DHN), a convolutional deep belief network (CDBN), a deconvolutional deep network (DDN), and the like, and preferably a CNN-based deep learning model, but the present application is not necessarily limited thereto.

In an embodiment, the modified CNN structure applied to the ECG encoder 12 in the present application is particularly well suited for ECG analysis, for the following reasons.

1) Use of squeeze excitation network: the squeeze excitation network ensures that the numerical vector extracted by the encoder effectively reflects the anatomical (positional) information of each channel, which improves quality of the encoder and the numerical vector obtained by the encoder. In an input of a multi-channel electrocardiogram, the input for each channel reflects a specific anatomical position of the heart. Since anatomical position information is crucial for diagnosing cardiac pathophysiology, in order to extract a number of clinically meaningful numerical features from the electrocardiogram, each numerical value needs to be nonlinearly synthesized by picking and choosing information that is suitable for a particular anatomical structure that each numerical value itself needs to reflect. The squeeze excitation enables representation by optimizing the degree of contribution for each channel through the aforementioned recalibration process when creating the representation that is served as the next layer. Specifically, when applied to the input terminal, feature extraction is performed to focus on a specific channel according to the anatomical structure, and when applied to the output terminal, abstract clinical information that is nonlinearly synthesized is selected.

2) Use a non-local neural network or non-local network: the non-local network allows the numerical vector extracted by the encoder to effectively reflect an interaction between temporally separated electrocardiogram features, thereby improving quality of the encoder and the numerical vector obtained by the encoder. When a clinical significance of an electrocardiogram waveform at a particular time point in an input of a single-channel or multi-channel electrocardiogram is interpreted, the electrocardiogram waveform before and after the particular time point should also be considered. Further, in order to apply this process of referencing and integrating information to distant waveform information, it is necessary to have a network that is capable of learning how suitable to integrate the distant information (features) into the information of the current position being interpreted, and the non-local network described above performs this role.

3) Use of a skip connection: the ECG encoder 12 of an embodiment of the present application uses a deep learning structure in which the input data is processed through multiple layers of nonlinear transformation. In this case, a gradient vanishing phenomenon may occur where the loss signal at the output terminal is not sufficiently transmitted to the input terminal. The skip connection effectively reduces this problem. In addition, the skip connection has an effect of improving quality of the numerical vector by allowing the information from the input terminal to be reflected at the output terminal with minimal deformation, so that the extracted numerical vector allows various features in the conversion process of the encoder to be widely reflected.

4) Multi-task learning: the multi-task learning is a way of training the encoder network with the network characteristics described above, which makes a single numerical vector obtained through the encoder commonly used in various downstream tasks during the training process, thus helping to make the numerical vector universal. As described above, the ECG encoder 12 of the present application outputs an abbreviated numerical vector of a fixed size and format through the embedding process, which is used to perform various downstream tasks. Here, the numerical vectors are used as input information for various machine learning algorithms for different purposes, and therefore a comprehensive clinical state of a patient should be extracted as efficiently as possible. In an embodiment of the present application, since an output vector of the ECG encoder 12 is configured to simultaneously perform multiple tasks to be described below, when the error signals from the output terminal of each downstream task network are back-propagated, the error signals converge to an end terminal of a single encoder to train the single encoder, and the encoder trained in this manner is capable of generating a universal embedding vector that achieves the above-mentioned purpose.

In an embodiment, the electrocardiogram data received by the ECG encoder 12 may include an electrocardiogram signal or an electrocardiogram image in which the electrocardiogram signal of all channels is illustrated on a two-dimensional plane.

In an embodiment, when the electrocardiogram data is an electrocardiogram signal, the electrocardiogram signal input to the ECG encoder 12 includes multi-channel or single-channel, and has the form of a two-dimensional array of $C \times M$ (the number of each input lead (channel) X the number of measurement values for each channel).

In an embodiment, when the electrocardiogram data is an electrocardiogram image, the electrocardiogram image is converted to an image containing all lead channels or an image for each lead channel cropped by each lead channel, such that the electrocardiogram image input to the ECG encoder 12 includes a black and white image of one or more lead channels or a color image consisting of R (Red), G (Green), and B (Blue) of three color channels, and is input in the form of a three-dimensional array of $C \times W \times H$ (the number of channels X the number of horizontal pixels X the number of vertical pixels).

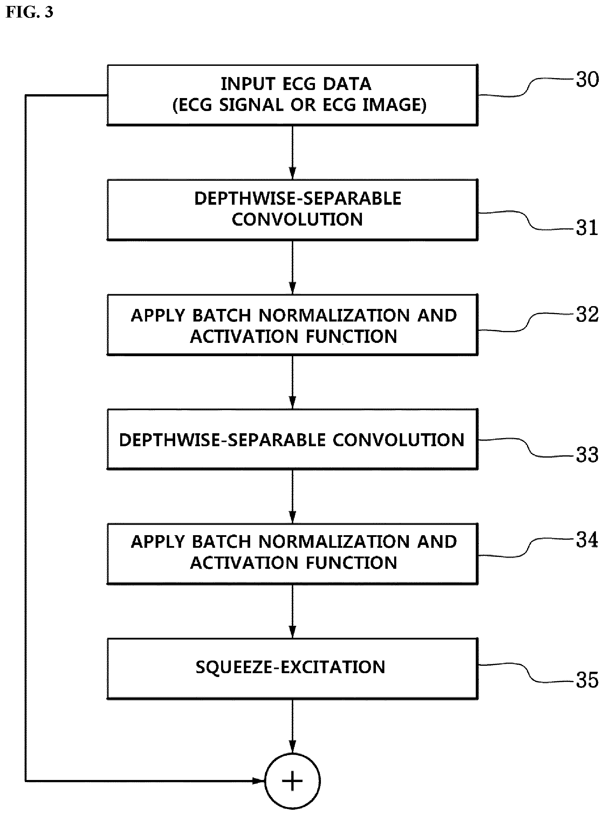

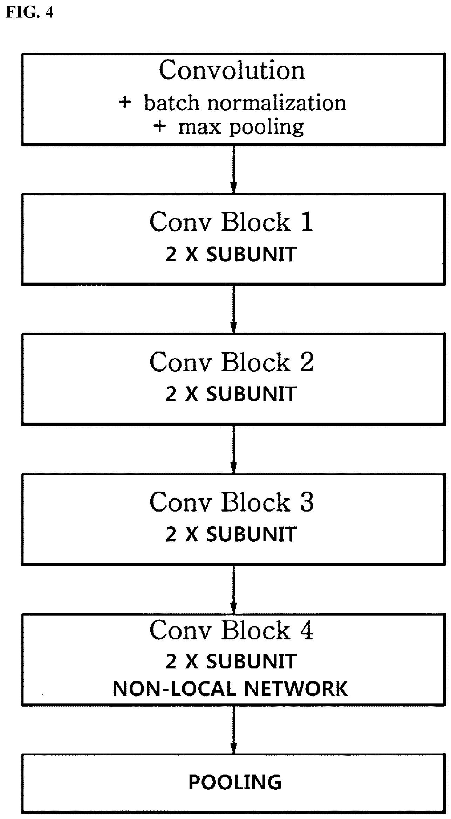

In an embodiment, the ECG encoder 12 may include a single convolution layer and a plurality of convolution blocks, in which each convolution block may include a plurality of consecutive ECG subunits. The ECG encoder 12 may convert the electrocardiogram data into a numerical vector by passing through a first convolution layer and a plurality of convolution blocks. With reference to FIGS. 3 and 4 below, a process in which the ECG encoder converts electrocardiogram data into a numerical vector is described in more detail.

In an embodiment, the analysis unit 14 performs the provision of information regarding disease-related analysis, prediction, or diagnosis using the numerical vector calculated by the ECG encoder 12.

Analysis results from the analysis unit 14 may include a disease prediction, and when the disease prediction is made, the electrocardiogram data is possible in both single-channel or multi-channel, and the disease may include shock, respiratory failure, pulmonary edema, cardiac arrest, cardiogenic syncope and sudden death, acute coronary syndrome, myocardial infarction, and hyperkalemia.

The analysis results from the analysis unit 14 may include a disease diagnosis, and when a disease is diagnosed, may include abnormalities in the rhythm of the heart (tachycardia, bradycardia, various arrhythmias) and abnormalities in the structure and function of the heart (heart failure, pericardial tamponade, stenosis/failure of valves, pulmonary arterial hypertension, pulmonary embolism, cardiomyopathy).

In an embodiment, the electrocardiogram data may be a plurality of electrocardiogram data measured at regular or irregular time intervals. The respective electrocardiogram data may be passed through the ECG encoder, and the respective numerical vectors may be acquired to respectively obtain a diagnosis from the analysis unit 14, or a plurality of numerical vectors may be simultaneously input to a machine learning algorithm to diagnose a disease or whether the disease improves or worsens.

The analysis unit 14 may arrange each of the numerical vectors obtained from the plurality of electrocardiogram data into a sequential vector. When processing a plurality of numerical vectors as input, the numerical vectors may be concatenated in a lengthwise direction of the vectors and converted to a single input and passed to a single multilayer perceptron (MLP) network, concatenated in a vertical direction of a length of the vector and passed to a single transformer network, or not concatenated and passed to a single recurrent neural network (RNN) sequentially according to the order in which tests are performed. The analysis unit 14 may also be learned to determine whether the patient has improved or worsened a particular disease over time based on a second numerical vector that has been passed through and extracted.

In an embodiment, the analysis unit 14 may also be learned to determine whether a particular disease has improved or worsened by further considering information on time.

A time interval between input vectors (e.g., the first numerical vector) may not be established. Then, the information on time, including the time interval, may be encoded using a separate function to calculate a value representing the time interval, and the information on time may be added to the input vector by using the vector in which the encoded value and the first numerical vector are concatenated as the input vector. Then, the analysis unit 14 may determine whether the disease has improved or worsened by further considering the time interval between the input vectors.

The information on the time may also be implemented as a separate function. This function may also be statistically modeled based on actual electrocardiogram data, or may be a function arbitrarily specified by a user. In this case, a function corresponding to the information on time is encoded and concatenated with the input vector.

In this case, the information on time may be encoded using the function and concatenated with each of the input numerical vectors to improve accuracy.

Meanwhile, in embodiments, the downstream processing unit 16 uses the numerical vector calculated from the ECG encoder to process a downstream task. In an embodiment, each task of the downstream tasks may be performed by a multi-layer perceptron (MLP) having two or more fully connected layers.

In an embodiment, a MLP network for each task may be trained together with the ECG encoder network, or may be trained separately after the ECG encoder 12 is trained first. When there are multiple downstream task networks, each task network is trained simultaneously through multi-task learning. The downstream task network may receive additional structured data input information in addition to the numerical vector output from the ECG encoder 12 to improve prediction accuracy. In this case, the additional structured data input information may be concatenated with the numerical vector or processed through a separate, different input network. The additional structured data input information may include at least one of age, gender, and an electrocardiogram and other vital signs, in which the electrocardiogram and other vital signs may include at least one of blood pressure, pulse rate, body temperature, respiratory rate, comorbidities, and oxygen saturation. In addition, structured numerical test results, unstructured test results with appropriate embedding (such as image tests), and unstructured textual data based on natural language may also be included as an additional input.

In an embodiment, the disease analysis apparatus 1 may be coupled with an electrocardiogram measurement device (e.g., a 12-lead electrocardiogram, electrocardiogram monitor, and other medical equipment using single-channel or multi-channel electrocardiogram) that is capable of directly analyzing electrocardiogram signals obtained retrospectively or prospectively from a patient, for example in real time, to provide clinical information.

As a non-limiting example, the disease analysis apparatus 1 may include, but is not limited to, a smartwatch, an exercise device such as a treadmill equipped with an electrocardiogram measurement device, and the like.

In addition, in an embodiment, the disease analysis apparatus 1 may be combined with an electrocardiogram interpretation equipment that provides clinical information with a local equipment or server by directly analyzing an image of a visualized electrocardiogram that has already been obtained and output to a paper or image.

As a non-limiting example, the disease analysis apparatus 1 may be, for example, an electronic health record (EHR) system that uses an app installed device, a camera or scanning device, and is equipped with an interpretation algorithm, but is not limited thereto.

Meanwhile, a method of providing auxiliary information regarding disease-related analysis, prediction, and diagnosis by converting electrocardiogram data into numerical vectors (hereinafter referred to as a "disease analysis method") is performed by a computing device including a processor. The computing device including the processor may be performed by, for example, the disease analysis apparatus 1 or at least some constituent elements thereof (e.g., the acquisition unit 10, the ECG encoder 12, the analysis unit 14, and/or the downstream task processing unit 16) [the downstream processing unit 16 may be present separately from the analysis unit 14 or included in the analysis unit 14], or may be performed by another computing device. Hereinafter, for clarity of description, the present application will be described in more detail with embodiments in which the disease analysis method is performed by the disease analysis apparatus 1.

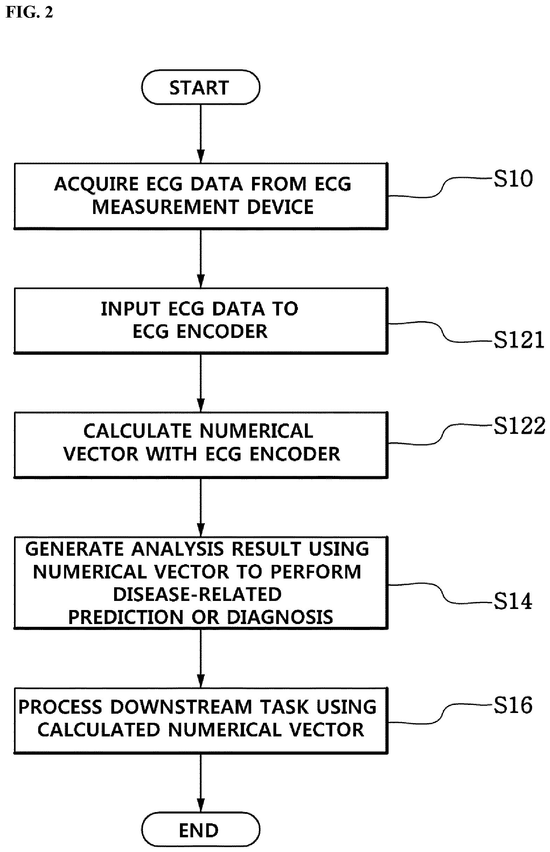

FIG. 2 is a flowchart of a disease analysis method according to an embodiment of the present application. With reference to FIG. 2, in a method of analyzing a disease from an electrocardiogram signal (ECG), performed by a processor, using deep learning, a disease analysis method further includes: acquiring (for example, by an acquisition unit 10) the electrocardiogram data from an electrocardiogram measurement device (S10); inputting the electrocardiogram data into an ECG encoder (e.g., by an ECG encoder 12) (S121); calculating a numerical vector using deep learning through the ECG encoder (S122); and (for example, by the analysis unit 14) performing to analyze, predict, or diagnose a disease using the numerical vector (S14); processing a downstream task using the numerical vector, for example by a downstream processing unit 16, in addition to or as part of the analysis step (S14) by the analysis unit 14 (S16), in which each task may be performed by a multi-layer perceptron (MLP) having two or more fully connected layers.

FIG. 3 is a view illustrating an ECG encoder subunit, according to an embodiment of the present application.

With reference to FIG. 3, in an embodiment, the ECG encoder 12 is based on CNN and includes a plurality of convolution blocks.

The ECG encoder 12 includes an ECG encoder subunit.
the ECG subunit is included in the remaining convolution blocks except for a first convolution layer.

The ECG encoder subunit may include a depthwise-separable convolution layer that independently convolves the electrocardiogram data for each channel.

In the ECG encoder subunit constituting the ECG encoder 12, the electrocardiogram data ( single-channel or multi-channel electrocardiogram signals or electrocardiogram images) passes through the depthwise-separable convolution layer two times and is input as input data to the next convolution layer through the skip connection. The depthwise-separable convolution is a form of depth-wise convolution followed by a point-wise convolution.

While a conventional convolution considers all channels and local information to create a single feature map, the depthwise- separable convolution calibrates the number of feature maps output through the depth-wise convolution and the point-wise convolution.

In an embodiment, the depth-wise convolution performs a convolution operation with an independent filter for each channel of the electrocardiogram data. The depth-wise convolution may also include a point-wise convolution operator that is directly connected to a depth-wise convolution operator. The depth-wise convolution separates $C \times W \times H$ input as units of channels and applies a convolution filter to each one to create an output, and then recombines the resulting outputs so that the convolution filter may create an output of the same size with much fewer parameters. It is advantageous when an operational result for each filter needs to be independent of other filters. The point-wise convolution is a filter referred to as a 1x1 Conv ($1\times$ for a one-dimensional input), which aims to shuffle the information for each channel out again. The method described above aims to reduce or increase a total number of channels. That is, the depth-wise convolution may generate output points in parallel by performing a convolution in a channel direction of the feature map. The point-wise convolution performs a convolution for each spatial point by concatenating the output values of independent channels through the depthwise-separable convolution. More than two output dimensions may be applied for each input channel to improve performance for each channel.

In an embodiment, after the depthwise-separable convolution, batch normalization may be performed. Learning efficiency is improved by making an input distribution for each layer constant. In addition to the batch normalization, various non-linear activation functions (e.g., ReLU), dropout, and the like may be used.

The ECG encoder subunit may include a squeeze-excitation layer in which an average is extracted for each channel to calculate a single scalar value, the scalar value for each channel is between 0 and 1, which is multiplied on each channel to recalibrate the values for each channel. This allows a deep learning algorithm to learn which channels are important.

The skip connection adds the existing input value in an element-wise manner to the resulting value from the above operations. If the number of channels in the output value has changed differently from the number of channels in the input value through the above operations, the existing input value is passed through a linear layer to calibrate the number of channels and then added to the output value above.

FIG. 4 is a view illustrating an ECG encoder according to an embodiment of the present application. With reference to FIG. 4, in an embodiment, the ECG encoder may include a single convolution layer at an input terminal and four subsequent convolution blocks. When input data is an electrocardiogram signal, the first convolution layer has 64 channel outputs with a typical 7x1 convolution. Then, after passing through a batch normalization layer and a max-pooling layer, the input data sequentially go through four convolution blocks. Each convolution block includes two sequential encoder subunits, and a final block may include a non-local network. When all blocks have been passed, in last, a global pooling process is performed. A kernel size (e.g., $7\times1$, $5\times1$, $3\times1$) for all convolution and pooling layers, a stride size, a padding method, and the number of output channels, as well as the number of blocks, the number of subunits for each block, and a placement of the non-local network, may all be determined appropriately based on the characteristics of the electrocardiogram signal.

In an embodiment, each encoder subunit may have a structure of a sequence of the depthwise-separable convolution layer (e.g., stride 2), the batch normalization layer, the depthwise-separable convolution layer (e.g., stride 1), the batch normalization layer, and the squeeze excitation layer, with reference to FIG. 3, and may include a single skip connection that performs an addition to a resulting vector by bypassing this sequence of processing.

A squeeze-excitation is a methodology where a key is a scaling by squeezing and recalibrating a feature map. Featured response for each channel is adaptively recalibrated by focusing on channel relationships and explicitly modeling interdependencies between channels.

In an embodiment, a squeeze operation may be applied when there is a conversion ($F_{tr} : X \rightarrow U$) that converts an input X with dimension [H', W', C'] into an output U with dimension [H, W, C].

For the output U, information for each channel is extracted ($F_{sq}$), and based on the information, a scalar weight for each channel is calculated ($F_{ex}$) and multiplied on the corresponding channel ($F_{scale}$). For convenience, assuming $F_{tr}$ is a convolution layer, a standard convolution learns a total of $C' \times C$ 2D filters when there are C' input channels and C output channels. A set of the learned filters is denoted by $V = \{v_1, v_2 ... v_c\}$ and a result of $F_{tr}$ is denoted by $U = \{u_1, u_2 ... u_c\}$.

The squeeze operation extracts information for each output channel to take channel dependency into account. That is, a scalar value z representing a channel is created using the entire channel information.

In an embodiment, in order for $F_{sq}$ to extract only important information from each channel, one of the most common methodologies, global average pooling, may be used. When the global average pooling is used, it has the advantage of not increasing parameters of a training target.

The squeeze operation may also be performed with the following equation.

$$z_c = F_{sq}(\mathbf{u}_c) = \frac{1}{H \times W} \sum_{i=1}^{H} \sum_{j=1}^{W} u_c(i,j)$$

Zc is a representative value for each channel. During the squeeze operation, a feature map with a size of $H \times W \times C$ is compressed to a size of $1 \times 1 \times C$ by the global average pooling operation. All the feature values corresponding to one channel in the feature map are added together and then divided by $H \times W$ to compress to $1 \times 1 \times 1$. Since the feature map has C channels, combining all the compression results for each channel results in data of size $1 \times 1 \times C$. As described above, each channel is compressed into one dimension, and the ECG feature values for each channel are added together to results in the representative value Zc for each channel. The resulting Zc vector is provided for the excitation operation.

After the important information has been squeezed out, a recalibration process is performed, referred to as an excitation operation, which calculates channel-wise dependencies. Two things are important when performing the excitation operation. First, a complicated relationship (non-linear) between channels needs to be found, and second, multiple channels need to be selected and emphasized, rather than just one of the C channels being weighted more heavily, as in a one-hot activation. To this end, in an embodiment, a fully connected layer and a nonlinear function need to be calibrated for this calculation.

A weight s, which is a parameter of the fully connected layer or nonlinear function, may also be represented by the following equation.

$$\mathbf{s} = F_{ex}(\mathbf{z}, \mathbf{W}) = \sigma(g(\mathbf{z}, \mathbf{W})) = \sigma(\mathbf{W}_2 \delta(\mathbf{W}_1 \mathbf{z}))$$

Here, s is a weight for each channel according to contribution of each ECG channel, with a value between 0 and 1. $\sigma$ is a sigmoid function, $\delta$ is RELU,

$$W_1 \in R^{\frac{C}{r} \times C},$$

$$\mathbf{W}_2 \in R^{C \times \frac{C}{r}}.$$

R is a reduction ratio, a hyperparameter to reduce the number of parameters in the fully connected layer through a dimension reduction. The recalibration operation may also be performed by multiplying the weight s for each channel.

A final numerical vector, Xc, is calculated that is recalibrated based on the weight s for each channel. This final numerical vector, Xc, may also be represented by the following equation

$$\tilde{\mathbf{x}}_c = F_{scale}(\mathbf{u}_c, s_c) = s_c \mathbf{u}_c$$

The finally calculated Xc is a vector, where each element Sc has a value between 0 and 1, which is applied as a scalar product to each of the corresponding channels and used to a weight for each channel. This allows a deep learning algorithm to learn which channels are important.

In an embodiment, a final convolution block of the encoder may further include a non-local network. The non-local network is to add an attention mechanism in a spatial manner. The attention mechanism obtains an inner product between a query vector for a particular spatial point in the feature map and a key vector for all spatial points. When the inner product value is normalized with a softmax operation, a scalar value corresponding to a weight between 0 and 1 may be obtained for each position in the feature map. This scalar value is multiplied by a value vector for each of the corresponding spatial points and added together, so that the value vector for a particular spatial point is converted into a weighted sum of the value vectors for all spatial points. The converted value is then combined with the original feature map through the skip connection to form an output value. The vectors calculated from the input feature map using each independent parameter function are used as the vectors corresponding to key, query, and value described above. When analyzing a feature at a particular time point in the electrocardiogram signal (which corresponds to a particular position in the one-dimensional input data), this process also allows signals from other time points that are farther away to be taken into account, enabling a more efficient determination of the overall context of the electrocardiogram data. In addition, when the input data is an image of a multi-channel waveform signal output in two dimensions, there is an additional benefit of being able to additionally refer to the waveforms of different channels.

In contrast, a typical CNN is limited to calculating only a local neighborhood. Even with an atrous convolution or a large kernel size, there is a limited area that the filter is capable of viewing at once. Similarly, a typical RNN has the limitation of only being able to consider only a limited time range of the immediate past. These operations that is able to only access local information along the time or spatial axis typically perform iterative operations to obtain a global view. However, these iterative operations are inefficient, difficult to be optimized, and often lead to the occurrence of multi-hop dependencies when modeling.

The present application overcomes the limitations of the aforementioned conventional CNN by using a non-local network, which allows for referencing features from all temporal positions in the feature map, as well as features from all different channels (in case of an image data to which an input is output), in the form of a weighted sum. In an embodiment of the present application, the non-local network was added to the final convolution block of the ECG encoder. However, the placement of the non-local network may vary depending on the input data and the intended use.

In an embodiment, one input data in which the electrocardiogram signal passes through a first convolution block to a fourth convolution block may be converted into a two-dimensional matrix of the form N (input size and spatial dimension of output, which is dependent on network structure) $\times$ D (channel size). This N $\times$ D matrix may be unfolded and converted to a single N $\times$ D dimensional vector, or may be pooled for each channel (e.g., global average pooling) and converted to a D dimensional vector to complete the entire encoding process of the electrocardiogram signal.

In a further embodiment, as an additional method of processing when the phase of each electrocardiogram signal does not match, the electrocardiograms stored in the medical record are often grouped in the manner of I, II, III / AVR, AVL, AVF / V1, V2, V3 / V4, V5, V6, with measurement time separated for each group. In this case, the phase within the group matches, but the phase between the groups does not match. Therefore, it is sometimes difficult to analyze all 12 channels with a single network at once. In embodiments of the present application, in this case, there is a separate ECG encoder network as described above for each lead group. Further, each output vector is combined by selecting and applying one of the concatenation, addition, or attention mechanisms into one embedding vector.

In a further embodiment, a plurality of different encoding networks trained with various settings can be collected and used together, such that the ECG encoder described above may use depthwise-separable convolution layers with various numbers and formats within each convolution layer, depending on the input signal, the problem being processed, and the equipment being analyzed. In addition, the kernel size, the stride size, the padding method, and the output size may be set variously for each convolution layer. In this case, it is possible to extract multiple embedding vectors from a single electrocardiogram signal, and these results can be synthesized (e.g., concatenation, addition, and attention mechanism) and used for disease prediction and diagnosis.

In an embodiment, when the input data is an electrocardiogram image, a basic configuration is the same as when the input data is an electrocardiogram signal, with the following exceptions. The input image is resized, cropped to a specific size for each channel, and normalized. The kernel of all convolution layers and depthwise-separable convolution layers are two-dimensional. The kernels of all pooling layers (max pooling and global average pooling) are two-dimensional. After a final pooling (e.g., global average pooling), the output is an N $\times$ D or D dimensional vector, which is the same as before. Since all channels are on one image without an input for each lead channel, there is no separate model for each lead (or lead group).

In an embodiment, the numerical vector values calculated from the ECG encoder can be used for the downstream task. Each task of the downstream tasks performs by a multi-layer perceptron (MLP) having two or more fully connected layers. The MLP for each task may be trained either 1) jointly with the ECG encoder, or 2) independently, receiving as input the embedding vector output by the ECG encoder 12 that has been trained first. When trained using method 2), only the downstream task MLP is trained with the weight values of the ECG encoder fixed, but after this training, a fine tuning process may be added in which the weight values fixation of the ECG encoder is released and the entire network is further trained through a backpropagation.

In an embodiment, the MLP for each task receives additional structured data input information that is different from the numerical vectors calculated by the ECG encoder 12 to improve prediction accuracy, and in this case, the additional input information may be concatenated with the numerical vectors after preprocessing such as standardization, or may be processed through a separate, different input network and then concatenated and processed as input.

In case of a multivariate regression problem (predicting various numerical values), the output numerical vectors are used as the output of the MLP.

In case of a classification problem (selecting one of several items), a probability of being included in each item is obtained by passing through a softmax function, and the item with the highest probability is selected.

In case that a problem of predicting whether a specific event occurs, each output value may be passed through a sigmoid function, which is interpreted as a probability that the event will occur. It can be seen that these probabilities are conditional probabilities obtained by interpreting the electrocardiogram data, and when these probabilities are output, a marginal probability that does not take the input electrocardiogram data into account is presented together as a baseline risk probability. A graph (e.g., a bar graph) may be displayed that visually shows how many times these probabilities have been increased by the electrocardiogram data (conditional probability/marginal probability).

In an embodiment, exemplary downstream tasks included in the present application are clinical diagnostic or predictive tasks, and includes: 1) a diagnosis of rhythmic abnormalities of the heart (tachycardia, bradycardia, various arrhythmias - sinus tachycardia, sinus bradycardia, atrial fibrillation/flutter, junctional rhythms, supraventricular tachycardia, blocks, ventricular tachycardia, and ventricular fibrillation), 2) a diagnosis of structural and functional abnormalities of the heart (heart failure, cardiac tamponade, narrowing/failure of valves - valvular stenosis, regurgitation, pulmonary hypertension, pulmonary embolism, and cardiomyopathy); and 3) a prediction of various clinical events (shock, respiratory failure, cardiac arrest, cardiac syncope and sudden cardiac arrest, acute coronary syndrome, myocardial infarction, ST elevation myocardial infarction, and hyperkalemia); and 4) a prediction of various clinical interventions (vasopressor infusion, ventilator application - mechanical ventilation, coronary angiography and interventions, and blood transfusion).

To this end, in addition to the electrocardiogram, additional structured data information may be received as input, and the additional structured data input corresponds to age, gender, and structured biometric information (blood pressure, pulse rate, respiratory rate, body temperature, numerical test results, etc.) and unstructured information that is structured through appropriate transformation (chief complaint, underlying disease, text, radiological and ultrasound imaging information, acoustic information such as auscultatory sounds, and various biosignals).

In embodiments of the present application, three main auxiliary learning tasks (supervised learning, self-supervised learning, and unsupervised learning) may be applied in the training process of the ECG encoder to improve the numerical vector (embedding) quality of the ECG encoder.

First, the supervised learning may be concurrently performed as the downstream task. The supervised learning is to perform tasks of calculating heart rate, pr interval, qrs duration, qt interval, corrected qt interval, p/qrs/t wave electrical axes, or determining whether the morphological features that correspond respectively to atrial fibrillation, atrial flutter, ectopic atrial rhythm, av block (first, second and third degree), left and right bundle branch block, intra-ventricular conduction delay, anterior and posterior fascicular block, ECG baseline wandering (baseline fluctuation noise), ECG signal artifact (signal noise), atrial premature beat complex (atrial premature contraction), ventricular premature beat complex (ventricular premature contraction), pacing rhythm (pacemaker rhythm), junctional rhythm (atrioventricular junctional rhythm), bigeminy, trigeminy, supra-ventricular rhythm, ventricular rhythm, left ventricular hypertrophy, right ventricular hypertrophy, ST elevation, ventricular repolarization, myocardial infarction, and q wave. The task based on the supervised learning improves quality of the numerical vector by ensuring that morphological characteristics of the electrocardiogram as described above, which are already well defined clinically, are reflected in the numerical vector.

For reference, the morphological characteristics mean morphological patterns that are repeatedly observed in the electrocardiogram waveform and have been extracted and defined by physicians or clinicians. The morphological characteristics are mostly independent of a position of the measured lead and may not be referred as a final diagnosis on their own.

Second, the self-supervised learning may be concurrently performed as the downstream task. The self-supervised learning includes, after transforming the original electrocardiogram data in a specific manner, 1) a method of inferring a type (and content) of the transformation, and 2) a method of restoring the original using the transformed input. The transformation used in method 1) in the above includes: i) adding noise to the original signal that follows a normal distribution; ii) changing a scale, where the original input is multiplied by a specific range of positive numbers; iii) inverting the waveform, where the original input is multiplied by -1; iv) time flipping, where the temporal placement of the original input is reversed; and v) interval shuffling, where the original data is divided into a certain range of time intervals and each interval is randomized. These transformations may be applied individually or in combination, and the main task is to determine which transformation (or combination) was applied. The model may be trained to infer the specific details (the amount of scale change or the order of interval shuffling) of the transformations depending on situations. The transformation used in method 2) in the above includes: i) adding noise that follows a normal distribution, ii) replacing a specific time interval with zero (restoring only the corresponding interval), and iii) adding a random non-linear curve (continuous and differentiable) to the original. In this case, the task is to infer the entire original or a part of the original (e.g., the original value of the interval replaced by zero in the above). The self-supervised learning task ensures that the numerical vectors better reflect the morphological characteristics of the electrocardiogram, allowing for the extraction of high-quality numerical vectors.

Third, the unsupervised learning may be concurrently performed as the downstream task. The unsupervised learning applied in the present application is as described below. The network training process of the present application, like the training of any other deep learning network, applies a data augmentation process that transforms the input data in a specific manner. In this process, N transformed electrocardiogram input data are created from a single electrocardiogram, which means that if there are M original electrocardiograms, M X N electrocardiogram input values are created. When two electrocardiograms are extracted from the M X N electrocardiograms, the numerical vectors created from this situation should be identical or very similar if the two electrocardiograms have the same original source. To satisfy this constraint, in the present unsupervised learning task, the following loss term is added to the existing loss function to minimize a distance between the two augmented data points from the same original source.

$$\beta \cdot I(VecA \text{ and } VecB \text{ is made from same } ECG \text{ signal}) \cdot \|VecA - VecB\|$$

Here, $\beta$ is a hyper-parameter that can be arbitrarily adjusted by a user input, I is an indicator function, and IIVecA-VecBII is a distance between two vectors created from two electrocardiograms. In an example, the distance may be measured using a Euclidean distance, but the present application is not limited thereto and the aforementioned distance factor may be changed, like the value of $\beta$, depending on each problem situation.

The addition of this loss term aims to train the encoder in such a way that each numerical vector are placed closer together in a vector space obtained from the numerical vectors as the numerical vector exhibit similar patterns. This encourages efficient placement of numerical vectors within the vector space defined by the numerical vectors, leading to an improvement in the embedding quality of the numerical vectors.

When concurrently performing auxiliary learning tasks based on the supervised, the self-supervised, or the unsupervised learning as described above, the downstream task network for learning is trained jointly with the ECG encoder network, which may be performed independently prior to the training of the downstream network for clinical diagnosis/prediction, or simultaneously with the training of the clinical diagnosis/prediction network. If a pretraining approach is followed, after completing the pretraining, the weights of the ECG encoder are fixed, and only the clinical diagnosis/prediction network is trained. Then, if necessary, the fixed weights of the ECG encoder is released, and a fine-tuning process is applied to simultaneously train both the encoder and the downstream task network for the clinical diagnosis/prediction. In case that the self-supervised learning network and clinical diagnosis/prediction network are trained simultaneously, weight updates occur across the entire weights of all networks, including the ECG encoder.

The pretraining and concurrent training of the above-mentioned supervised, self-supervised, and unsupervised learning increases the universality of the numerical vectors (embedding vectors) output by the ECG encoder by ensuring that the vectors simultaneously add and include morphological information corresponding to the clinician-defined patterns seen in the electrocardiogram and its own unrelated morphological information (supervised/self-supervised learning). Also, by efficiently rearranging the vector space in which the numerical vectors are placed (unsupervised learning), the ECG encoder can be efficiently used for other types of downstream tasks that are not initially planned. That is, there is a further benefit to implementing few-shot, one-shot learning.

Meanwhile, in the following embodiments, examples of applications of the aforementioned ECG encoder or numerical vectors extracted therefrom are described below.

## Example of application of numerical vector

An exemplary application of the exemplary numerical vectors of the present application is the diagnosis and triage of patients in clinical practice, emergency, and disaster sites: In addition to the numerical vector obtained from the ECG encoder, additional information may be concatenated to form a single input vector, which may be passed through a new downstream task network and used to perform a desired clinical diagnosis, clinical event/treatment prediction.

The additional structured data information may include: at least one of age, gender, or vital signs such as blood pressure, pulse rate, body temperature, respiratory rate, and oxygen saturation; conventional structured information such as various numerical laboratory test results; unstructured data (images, sounds, biosignals, etc.) transformed into structured numerical information through machine learning methods; and natural language information such as symptoms, diagnoses, medical records, etc. transformed into numerical vectors through natural language processing.

The downstream task network used may preferably be the batch normalization already mentioned above, a dropout layer, and a non-linear activation function (e.g., a multilayer perceptron neural network consisting of two or more fully-connected layers with Relu), but the specific configuration may vary depending on the intended use.

When training a new downstream task network, first, as described above, the weights of the ECG encoder may be fixed, and then the weights of the new downstream network may be updated through training, followed by fine tuning, where the entire weight of the ECG encoder and downstream task network is updated through additional training.

Another application of the exemplary numerical vectors of the present application includes applications in electrocardiograms, intensive care monitors, and various healthcare equipment. The ECG encoder or the numerical vectors extracted therefrom can be used in various healthcare equipment (smartwatches, exercise machines, etc.). In addition to numerical vectors, these healthcare equipment may receive additional information as input in the same way as the applications described above.

For example, in case of an encoder used in healthcare equipment, the encoder is designed to receive input data of single-channel to four-channel electrocardiogram signals due to its nature. Since various healthcare equipment is connected to a patient and needs to process continuous streams of data from the patient, the signal from the patient can be divided into regular time intervals (e.g., 5 seconds), and each divided data interval can be analyzed using the ECG encoder and the downstream task network to provide diagnostic or predictive services based on the analysis results at each time point or a weighted average at each time point of the analysis results at each time point. The diagnostic or predictive target of such healthcare equipment is the diagnostic or predictive information desired to be obtained by the healthcare equipment, which may include, for example, a user's deterioration or various emergencies at rest and/or under exercise/drug load, which may include arrhythmias, angina, pulmonary edema, pulmonary embolism, acute coronary syndrome, electrolyte abnormalities, etc.

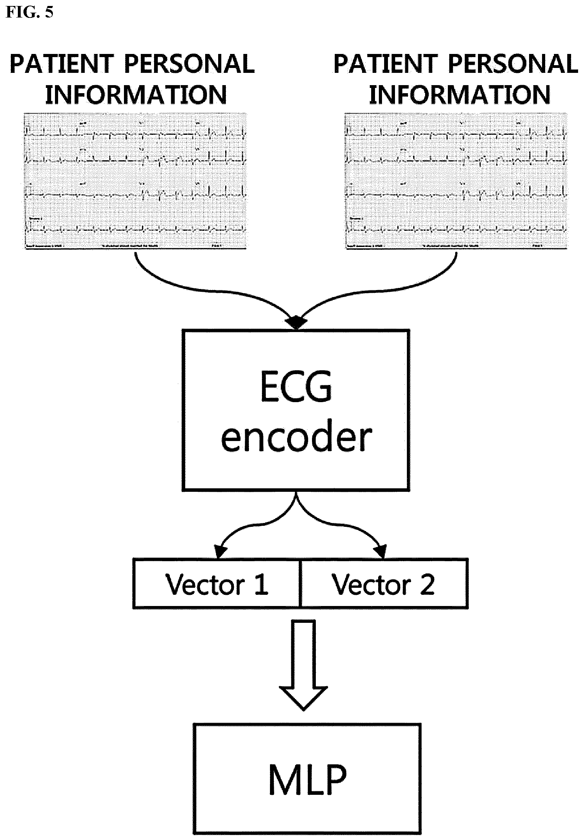

FIG. 5 is a view illustrating a utilization of numerical vectors obtained from a plurality of electrocardiogram data obtained through repeated measurements, according to another embodiment of the present application.

With reference to FIG. 5, the electrocardiogram is often performed multiple times on a single patient. The electrocardiogram is performed every few minutes to hours in suspected acute coronary syndromes and every few weeks to years in stable patients. The idea behind these repeated measurements is that the morphological changes in the electrocardiogram over time are clinically evaluated by doctors to diagnose the risk of specific diseases/conditions. To achieve the same functionality through artificial intelligence, the unstructured morphological features of each electrocardiogram data obtained from repeated trials must be quantified in a consistent manner, which is performed by the ECG encoder of the embodiment of the present application.

That is, first, by using a method that analyzes two electrocardiograms that meet specific clinical standards (e.g., time intervals), two numerical vectors obtained by passing respective electrocardiogram data through respective ECG encoders (the two ECG encoders may have the same parameter weights: parameter sharing) are concatenated to form a single input numerical vector. Further, a network having the same structure as the aforementioned downstream task network is created and the diagnosis to be predicted is set as an output value to train the model. Examples of applications in this case include, but are not limited to, acute coronary syndrome, adverse drug reactions after long-term administration of anticancer drugs (development of heart failure), improvement (improvement of shock) or deterioration (development of heart failure/pulmonary edema) of a patient's condition before and after fluid therapy.

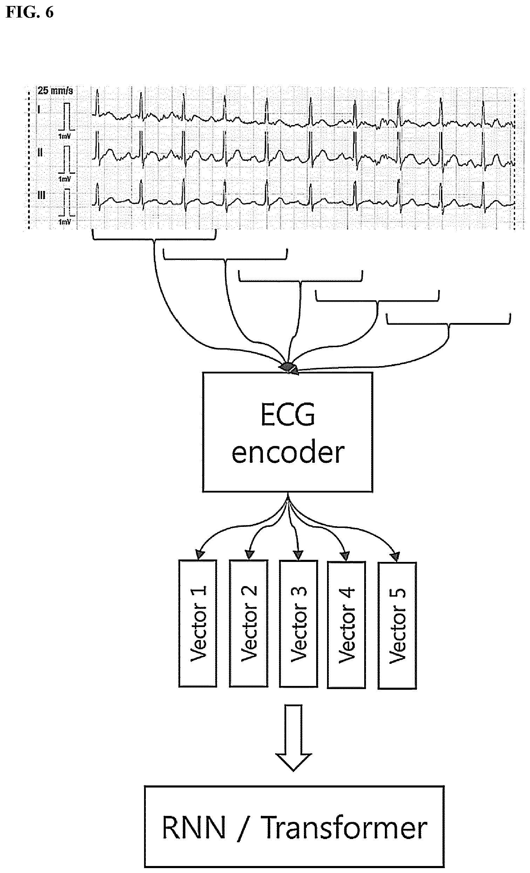

FIG. 6 is a view illustrating a utilization of N sequentially obtained numerical vectors, according to still another embodiment of the present application.

With reference to FIG. 6, N sequentially performed electrocardiograms that meet specific clinical standards are passed through the single ECG encoder 12. This corresponds to the embedding of electrocardiogram data, which is unstructured data, to obtain N sequentially obtained numerical vectors. After the obtained sequential embedding vectors are taken as input, a learning model may be trained and used to predict whether a patient will improve or worsen a specific disease over time, or whether a specific clinical event will occur by passing the vectors through a typical RNN (LSTM or GRU) or transformer network. Each sequential numerical vector used as input may be concatenated and augmented with additional information, which may include clinical information (age, gender, blood pressure, pulse rate, respiratory rate, temperature, symptoms, structured test results) converted to numerical vectors. The RNN or transformer network used herein is just one example of a neural network structure that is capable of processing sequentially constructed numerical vectors with repeated measurements, but any machine learning algorithm that is capable of performing similar functions can be used.

For an example of application, by using multiple 12-lead electrocardiograms that have been measured repeatedly, an artificial intelligence algorithm can be embedded in a 12-lead electrocardiogram machine or electronic medical record program to calculate the risk of acute coronary syndrome, pulmonary edema, pulmonary embolism, respiratory failure, cardiac arrest, and hyper- or hypokalemia.

As an example of another application, there is intensive care monitor devices and server equipment equipped with algorithms that receives, as sequential input, a patient's electrocardiogram information measured through one or more channels in real time and cut into segments of a certain length (temporal duration: e.g., 5 seconds), calculates the risk of acute coronary syndrome, pulmonary edema, pulmonary embolism, respiratory failure, cardiac arrest, and hyper- or hypokalemia in real time, and alert the user If the risk increases above a certain level, but the present application is not limited thereto.

The disease analysis apparatus described above may be implemented by a computing device including at least some of a processor, a memory, a user input device, and a presentation device.

The memory is a medium for storing computer-readable software, applications, program modules, routines, instructions, and/or data that is coded to perform specific tasks when executed by a processor. The processor may read and execute computer-readable software, applications, program modules, routines, instructions, and/or data stored in memory. The user input device may be a means for the user to enter instructions to the processor to execute a specific task, or to enter data necessary for the execution of the specific task. The user input device may include a physical or virtual keyboard or keypad, key buttons, mouse, joystick, trackball, touch-sensitive input means, microphone, or the like. The presentation device may include a display, printer, speaker, or vibrating device.

The computing device may include a variety of devices such as smartphones, tablets, laptops, desktops, servers, and clients. The computing device may be a single, standalone device or may include multiple computing devices operating in a distributed environment consisting of multiple computing devices that cooperate with each other over a communication network.

In addition, the disease analysis method described above may be executed by a computing device having a processor and also having a memory storing computer-readable software, applications, program modules, routines, instructions, and/or data structures coded to be executed by the processor to perform the disease analysis method by converting electrocardiogram data into numerical vectors.

The above-mentioned embodiments of the present embodiments may be implemented by various means. For example, the present embodiments may be implemented by hardware, firmware, software, or a combination thereof.

In the case of the implementation by hardware, the method of analyzing a disease according to the present embodiments may be implemented by one or more of application specific integrated circuits (ASICs), digital signal processors (DSPs), digital signal processing devices (DSPDs), programmable logic devices (PLDs), field programmable gate arrays (FPGAs), processors, controllers, micro-controllers, microprocessor, and the like.

For example, the method of analyzing disease by converting electrocardiogram data into numerical vectors according to embodiments may be implemented using an artificial intelligence semiconductor device in which the neurons and synapses of a deep neural network are implemented as semiconductor devices. In this case, the semiconductor device may be a currently used semiconductor device, such as SRAM, DRAM, NAND, etc. or a next-generation semiconductor device, such as RRA, STT MRAM, PRAM, etc. or a combination thereof.

When the method of analyzing disease by converting electrocardiogram data into numerical vectors according to embodiments is implemented using the artificial intelligence semiconductor device, the results (weights) of training the neural network model with software may be transferred to synapse mimicking elements placed in an array, or training may be performed on the artificial intelligence semiconductor device.

In case of implementation by firmware or software, the method of analyzing disease by converting electrocardiogram data into numerical vectors according to the present embodiments may be implemented in the form of an apparatus, procedure, or function that performs the functions or operations described above. A software code may be stored in a memory unit and executed by a processor. The memory unit is positioned inside and outside the processor and may transmit and receive data to/from the processor by means of various means already well known.

Further, as described above, terms such as "part," "device," "module," "system," "processor," "controller," "component," "interface," or "unit" may generally refer to computer-related entity hardware, a combination of hardware and software, software, or executing software. For example, the constituent elements described above may be, but are not limited to, a process that is driven by a processor, a processor, a controller, a control processor, an object, an executable thread, a program, and/or a computer. For example, all of the applications running on the controller or the processor and the controller or processor can be constituent elements. One or more constituent elements may be within a process and/or an execution thread, and the constituent elements may be located on a single device (e.g., a system, computing device, etc.) or located to be distributed across two or more devices.

The above description is simply given for illustratively describing the technical spirit of the present application, and those skilled in the art to which the present application pertains will appreciate that various changes and modifications are possible without departing from the essential characteristic of the present technical ideas. In addition, the embodiments are provided for illustrative purposes only but are not intended to limit the technical concept of the present application. The scope of the technical concept of the present application is not limited thereto. The protective scope of the present application should be construed based on the following claims, and all the technical spirit in the equivalent scope thereto should be construed as falling within the scope of the present application.

Claims

1. An apparatus for converting electrical biosignal data into numerical vectors comprising:

an acquisition unit configured to acquire electrical biosignal data; and
an encoder configured to calculate a first numerical vector by receiving the electrical biosignal data using a deep learning algorithm,

wherein the first numerical vector is structured data associated with features extracted from the electrical biosignal data, including at least one of anatomical features and temporal features that are capable of being extracted from the electrical biosignal data.

2. The apparatus of claim 1, wherein the apparatus comprises a plurality of downstream processing units configured to simultaneously process a plurality of downstream tasks using the first numerical vector as an input numerical vector, and
wherein error signals from each of downstream task network output terminals are back-propagated and gathered at an end terminal of the encoder to train the encoder to improve universality in the first numerical vector.

3. The apparatus of claim 2, wherein the first numerical vector is concatenated with itself or with other structured data information to be used as the input numerical vector of the downstream processing unit.

4. The apparatus of claim 2, wherein the encoder is two or more, and wherein a plurality of first numerical vectors output from each encoder are concatenated to provide a single input numerical vector.

5. The apparatus of claim 2, wherein N sequential electrical biosignal data is passed through a single encoder to provide N sequential first numerical vectors.

6. The apparatus of claim 2, wherein the apparatus provides an analysis, diagnosis, or prediction of a particular disease based on result values for each time point obtained by dividing the electrical biosignal data into certain time intervals and passing information from each divided data section through the encoder or the encoder and a downstream processing unit, or a weighted average for each time point of the corresponding result values for each time point.

7. The apparatus of claim 2, wherein the apparatus fixes a network weight of the encoder in training a network of the downstream task and then modifies a network weight of the downstream task through the training, and further modifies an entire weight of the network of the encoder and the network of the downstream task through additional training.

8. The apparatus of claim 2, wherein each of the plurality of downstream processing units is performed by a multi-layer perceptron (MLP) having two or more fully connected layers.

9. The apparatus of claim 8, wherein the MLP is trained through a multi-task learning jointly with an encoding network training of the encoder, or is trained separately after the encoder is trained first.

10. The apparatus of claim 8, wherein the MLP receives additional structured data input information that is different from the first numerical vector,

wherein the additional structured data input information comprises at least one of age, gender, biosignals, numerical test results, natural language based structured data information converted into structured data through natural language processing, and structured data information converted from a different kind of biosignal data than the electrical biosignal data acquired from an acquisition unit,
wherein the biosignals comprise one or more biosignals selected from a group consisting of blood pressure, pulse rate, body temperature, respiratory rate, and oxygen saturation, and
wherein the additional structured data entry information is either concatenated with the first numerical vector or input separately from the first numerical vector.

11. The apparatus of claim 8, further comprising:
a display unit configured to representing marginal probability of a particular disease occurrence in consideration of the acquired electrical biosignal data, and marginal probability of a particular disease occurrence not in consideration of the acquired electrical biosignal data when outputting the MLP, and configured to displaying a ratio of an increase in the marginal probability of a particular disease occurrence in consideration of the acquired electrical biosignal data to the marginal probability of a particular disease occurrence not in consideration of the acquired electrical biosignal data, in case that the MLP predicts whether a particular disease occurs.

12. The apparatus of claim 1, wherein the deep learning algorithm of the encoder is based on CNN,

wherein the apparatus comprises an encoder subunit,

wherein the encoder subunit comprises:

one or more convolution layers;
one or more fully connected layers, where the fully connected layer comprises a nonlinear activation function; and
a concentration layer configured to summarize feature sets extracted from the electrical biosignal data per channel to extract a representative value for each, and recalibrate the feature set per channel to reflect contribution of the feature set per channel on the basis of the representative values,
wherein the feature set comprises features related to anatomical structures for each channel, and
wherein the recalibrated feature set per channel is more focused on features related to anatomical structures for each channel compared to the feature set.

13. The apparatus of claim 12, wherein the one or more convolution layers comprise a depthwise-separable convolution layer configured to individually perform a convolution of electrical biosignal data for each of the one or more channels.

14. The apparatus of claim 12, wherein the concentration layer performs a pooling process on the feature set for summarizing the feature set.

15. The apparatus of claim 12, wherein the concentration layer passes the representative value for each channel through the fully connected layer to calculate a contribution for each channel, and
wherein the contribution for each channel is multiplied by the feature set to recalibrate the feature set for each channel.

16. The apparatus of claim 15, wherein the concentration layer calculates the contribution per each channel by scaling a result of passing the representative value per each channel through the fully connected layer to a numerical value between a specific range.

17. The apparatus of claim 12, wherein the encoder comprises a plurality of convolution blocks, and
wherein the subunits are comprised in a remaining convolution blocks except for a first conversion layer.

18. The apparatus of claim 17, wherein the convolution block comprises:

a first encoder subunit; and
a second encoder subunit,
wherein the first encoder subunit is applied closer to an input terminal of the convolution block than an output terminal thereof compared to the second encoder subunit, and the concentration layer focuses more on the operation of summarizing the feature set to extract the representative value between the operation of summarizing the feature set extract the representative value and the operation of recalibrating according to the contribution of each channel compared to the second encoder subunit. - the features related to the anatomical structure are reflected more in the representative values of the first encoder subunit compared to the representative values of the second encoder subunit, and
wherein the second encoder subunit is applied closer to the output terminal of the convolution block than the input terminal thereof compared to the first encoder subunit, and the concentration layer focuses more on the operation of recalibrating according to the contribution of each channel compared to the first encoder subunit between the operation of summarizing the feature set to extract the representative value and the operation of recalibrating according to the contribution of each channel.

19. The apparatus of claim 17, wherein a final convolution block of the encoder further comprises a non-local network, and
wherein the non-local network implements spatial attention by comparing similarity between spatial points of the electrical biosignal data.

20. The apparatus of claim 1, wherein the electrical biosignal data is a signal of a single-channel or multi-channel, and
wherein the electrical biosignal signals input to the encoder are in the form of a two-dimensional array of $C \times M$ (the number of each input lead (channel) X the number of measured values for each channel).

21. The apparatus of claim 1, wherein the electrical biosignal data is an electrical biosignal image,

wherein the electrical biosignal image depicts the electrical biosignal signals of all channels on a two-dimensional plane,

wherein the electrical biosignal image is converted to an image that includes all of the lead channels or to an image per lead channel that is cropped per lead channel, and

wherein the electrical biosignal image input to the encoder is converted into a black-and-white image of one or more lead channels or a three-dimensional array of C × W × H (number of channels × number of horizontal pixels × number of vertical pixels) with three channels of R(Red), G(Green), and B(Blue) and input to the encoder.

22. The apparatus of claim 1, further comprising:
an analysis unit configured to analyze, predict, or provide diagnostic auxiliary information related to a disease or health using the first numerical vector.

23. The apparatus of claim 22, wherein the analysis results include a disease prediction,

wherein the electrical biosignal data is the electrical biosignal data of the single-channel, when the analysis unit predicts disease, and

wherein the disease includes shock, respiratory failure, cardiac arrest, acute coronary syndrome, myocardial infarction, and hyperkalemia, when the electrical biosignal data is electrocardiogram (ECG) data.

24. The apparatus of claim 22, wherein the analysis results comprises disease diagnostic auxiliary information to determine whether a disease has improved or worsened using the first numerical vector,

wherein the electrical biosignal data is a plurality of electrical biosignal data measured at regular intervals when the analysis unit provides the disease diagnostic auxiliary information, and

wherein each of the plurality of electrical biosignal data passes through a pooling layer of the encoder to provide the diagnostic auxiliary information.

25. The apparatus of claim 22, wherein the analysis results comprises a provision of the disease diagnostic auxiliary information,

wherein the electrical biosignal data is a plurality of electrical biosignal data measured at regular or irregular time intervals, and

wherein the analysis unit is trained to determine whether a particular disease has improved or worsened over time by arranging each of the first numerical vectors of the plurality of electrical biosignal data into sequential vectors, and concatenating the sequential vectors in a lengthwise direction of the vectors to pass the sequential vectors through the multilayer perceptron (MLP) network, or concatenating the sequential vectors in a vertical direction of the vector length to pass the sequential vectors through a transformer network, or sequentially passing the sequential vectors through an RNN without concatenating the sequential vectors to extract a second numerical vector.

26. The apparatus of claim 25, wherein before the sequential vectors are input for training, information on time is encoded, the encoded time information value is concatenated with the first numerical vector, and the analysis unit is trained by inputting the concatenated vector as a sequential vector.

27. The apparatus of claim 1, wherein the network of the encoder is trained through a self-supervised learning on the basis of clinically defined morphological characteristics among characteristics of the electrical biosignal data.

28. The apparatus of claim 1, wherein the network of encoder is trained by a self-supervised learning using electrical biosignal data transformed in a particular way as training data.

29. The apparatus of claim 1, wherein the network of the encoder is trained by an unsupervised learning, using the augmented electrical biosignal data as training data, and

wherein the encoder network comprises a process of inputting each of the augmented electrical biosignal data having the original electrical biosignal data in common to the encoder, and calibrating each of the calculated first numerical vectors to be identical or have a high degree of similarity.

30. The apparatus of claim 29, wherein the process of calibrating each of the calculated first numerical vectors to be identical or have a high degree of similarity is to minimize a distance of each of the calculated first numerical vectors.

31. The apparatus of claim 1, wherein the apparatus is concatenated with a smart watch, a medical device or exercise equipment equipped with an electrical biosignal measurement device or a device with a smartphone app or an electronic health record system.

32. The apparatus of any one of claims 1 to 31, wherein the electrical biosignal is an electrocardiogram (ECG).

**FIG. 1**

FIG. 2

```
                        ┌─────────────┐
                        │    START    │
                        └─────────────┘
                               │
                               ▼
        ┌──────────────────────────────────────┐
        │   ACQUIRE ECG DATA FROM ECG          │──────S10
        │   MEASUREMENT DEVICE                 │
        └──────────────────────────────────────┘
                               │
                               ▼
        ┌──────────────────────────────────────┐
        │        INPUT ECG DATA TO             │
        │        ECG ENCODER                   │──────S121
        └──────────────────────────────────────┘
                               │
                               ▼
        ┌──────────────────────────────────────┐
        │      CALCULATE NUMERICAL             │──────S122
        │   VECTOR WITH ECG ENCODER            │
        └──────────────────────────────────────┘
                               │
                               ▼
        ┌──────────────────────────────────────┐
        │  GENERATE ANALYSIS RESULT USING      │
        │  NUMERICAL VECTOR TO PERFORM         │
        │  DISEASE-RELATED                     │──────S14
        │  PREDICTION OR DIAGNOSIS             │
        └──────────────────────────────────────┘
                               │
                               ▼
        ┌──────────────────────────────────────┐
        │ PROCESS DOWNSTREAM TASK USING        │──────S16
        │ CALCULATED NUMERICAL VECTOR          │
        └──────────────────────────────────────┘
                               │
                               ▼
                        ┌─────────────┐
                        │     END     │
                        └─────────────┘
```

**FIG. 3**

```
INPUT ECG DATA
(ECG SIGNAL OR ECG IMAGE)        ──30

        │
        ▼

DEPTHWISE-SEPARABLE
CONVOLUTION                      ──31

        │
        ▼

APPLY BATCH NORMALIZATION AND    ──32
ACTIVATION FUNCTION

        │
        ▼

DEPTHWISE-SEPARABLE CONVOLUTION  ──33

        │
        ▼

APPLY BATCH NORMALIZATION AND    ──34
ACTIVATION FUNCTION

        │
        ▼

SQUEEZE-EXCITATION               ──35

        │
        ▼
       (+)
```

**FIG. 4**

```
┌─────────────────────────────────┐
│         Convolution             │
│    + batch normalization        │
│    + max pooling                │
└─────────────────────────────────┘
              │
              ▼
┌─────────────────────────────────┐
│        Conv Block 1             │
│        2 X SUBUNIT              │
└─────────────────────────────────┘
              │
              ▼
┌─────────────────────────────────┐
│        Conv Block 2             │
│        2 X SUBUNIT              │
└─────────────────────────────────┘
              │
              ▼
┌─────────────────────────────────┐
│        Conv Block 3             │
│        2 X SUBUNIT              │
└─────────────────────────────────┘
              │
              ▼
┌─────────────────────────────────┐
│        Conv Block 4             │
│        2 X SUBUNIT              │
│      NON-LOCAL NETWORK          │
└─────────────────────────────────┘
              │
              ▼
┌─────────────────────────────────┐
│           POOLING               │
└─────────────────────────────────┘
```

FIG. 5

PATIENT PERSONAL
INFORMATION

PATIENT PERSONAL
INFORMATION

ECG
encoder

| Vector 1 | Vector 2 |
|----------|----------|

MLP

**FIG. 6**

**INTERNATIONAL SEARCH REPORT**

International application No.

**PCT/KR2021/020227**

| A. | CLASSIFICATION OF SUBJECT MATTER |
|---|---|

**A61B 5/346**(2021.01)i; **A61B 5/327**(2021.01)i; **A61B 5/00**(2006.01)i; **G16H 50/20**(2018.01)i; **G06N 3/08**(2006.01)i; **G06N 3/04**(2006.01)i

According to International Patent Classification (IPC) or to both national classification and IPC

| B. | FIELDS SEARCHED |
|---|---|

Minimum documentation searched (classification system followed by classification symbols)

A61B 5/346(2021.01); A61B 5/00(2006.01); A61B 5/021(2006.01); A61B 5/0402(2006.01); A61B 5/24(2021.01); G06N 20/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 바이오 (bio), 신호 (signal), 데이터 (data), 벡터 (vector), 인코더 (encoder), 딥러닝 (deep learning), 심전도 (ECG)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2020-0068161 A (KONYANG UNIVERSITY INDUSTRIAL COOPERATION GROUP) 15 June 2020 (2020-06-15)<br>See paragraphs [0016], [0055], [0076], [0097] and [0130]; and claims 1 and 4. | 1,12-32 |
| A | | 2-11 |
| Y | KR 10-2020-0017709 A (INDUSTRY-ACADEMIC COOPERATION FOUNDATION, YONSEI UNIVERSITY) 19 February 2020 (2020-02-19)<br>See paragraph [0041]. | 1,12-32 |
| A | KR 10-2057047 B1 (KOREA INSTITUTE OF SCIENCE & TECHNOLOGY INFORMATION) 18 December 2019 (2019-12-18)<br>See entire document. | 1-32 |
| A | KR 10-2020-0072154 A (INDUSTRY-ACADEMIC COOPERATION FOUNDATION, YONSEI UNIVERSITY) 22 June 2020 (2020-06-22)<br>See entire document. | 1-32 |

✓ Further documents are listed in the continuation of Box C.    ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 April 2022** | **11 April 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/020227** |

**C.     DOCUMENTS CONSIDERED TO BE RELEVANT**

| Category* | Citation of document, with indication, where appropriate, of the relevant passages | Relevant to claim No. |
| --- | --- | --- |
| A | KR 10-2019-0011026 A (INBODY CO., LTD. et al.) 01 February 2019 (2019-02-01)<br>See entire document. | 1-32 |

Form PCT/ISA/210 (second sheet) (July 2019)

**INTERNATIONAL SEARCH REPORT**
Information on patent family members

International application No.

**PCT/KR2021/020227**

| Patent document cited in search report | | | Publication date (day/month/year) | Patent family member(s) | | | Publication date (day/month/year) |
|---|---|---|---|---|---|---|---|
| KR | 10-2020-0068161 | A | 15 June 2020 | KR | 10-2226875 | B1 | 11 March 2021 |
| KR | 10-2020-0017709 | A | 19 February 2020 | KR | 10-2174232 | B1 | 04 November 2020 |
| KR | 10-2057047 | B1 | 18 December 2019 | US | 2020-0268270 | A1 | 27 August 2020 |
| KR | 10-2020-0072154 | A | 22 June 2020 | None | | | |
| KR | 10-2019-0011026 | A | 01 February 2019 | None | | | |

Form PCT/ISA/210 (patent family annex) (July 2019)